# EUROPÄISCHE PATENTSCHRIFT

(11) **EP 1 940 905 B1**
(45) Veröffentlichungstag und Bekanntmachung des Hinweises auf die Patenterteilung: **11.08.2010**
(21) Anmeldenummer: 06806814.7
(22) Anmeldetag: 26.09.2006
(51) Int. Cl.: C08G 63/00

(54) **PRÄPARATE UMFASSEND HYPERVERZWEIGTE POLYMERE**
PREPARATIONS CONTAINING HYPERBRANCHED POLYMERS
PREPARATION COMPRENANT DES POLYMERES HYPERRAMIFIES

(30) Priorität: 25.10.2005 DE 102005051334; 26.08.2006 DE 102006040123
(43) Veröffentlichungstag der Anmeldung: 09.07.2008
(73) Patentinhaber: Evonik Degussa GmbH, 45128 Essen (DE)
(72) Erfinder: SEILER, Matthias, 64347 Griesheim (DE); KOBUS, Axel, 63225 Langen (DE)
(86) Internationale Anmeldenummer: PCT/EP2006/066722
(87) Internationale Veröffentlichungsnummer: WO 2007/048672

(56) Entgegenhaltungen:
- EP-A2- 1 557 441
- WO-A2-2006/038110
- US-B1- 6 379 683

## Beschreibung

Die vorliegende Erfindung betrifft Präparate umfassend mindestens eine niedermolekulare Substanz und mindestens ein hyperverzweigtes Polymer.

Präparate von hyperverzweigten Polymeren sowie niedermolekularen Substanzen sind an sich bekannt. Im Allgemeinen werden derartige Präparate eingesetzt, um eine verzögerte Wirkung der niedermolekularen Substanz zu erzielen. Des Weiteren weisen derartige Präparate vielerlei positive Eigenschaften auf.

Hyperverzweigte Polymere sind vielfach beschrieben. Beispielsweise sind derartige Polymere in der Druckschrift EP-A-0630389 dargelegt. Hierbei werden insbesondere hyperverzweigte Polyester beschrieben, die in Acrylharzen, gesättigten Polyester- und ungesättigten Polyesterharzen, Epoxidharzen, Phenolharzen, Polyurethanharzen oder Dentalmaterialien eingesetzt werden können. Eine Verwendung dieser Polymere als Trägerstoff, um niedermolekulare Substanzen verzögert freizusetzen, wird hierin nicht beschrieben.

Darüber hinaus werden hyperverzweigte Polymere in WO 97/06825 dargelegt. Die hierin beschriebenen hyperverzweigten Polymere stellen vielfach Polyamide dar, die in einer Vielzahl von Anwendungen eingesetzt werden können. Wesentlich ist hierbei, dass das Initiatormolekül eine aromatische Verbindung darstellt, die in einem einzigen Prozessschritt zu einem hyperverzweigten, Polymer umgesetzt werden kann. Die so erhaltenen hyperverzweigten Polymere können in einer Vielzahl von Anwendungen eingesetzt werden. Hierzu gehören insbesondere Konstruktionskunststoffe, Fasern, Folien, Kunststoffe zur Herstellung von Flaschen, ein Träger für Arzneimittel, sowie Membranen und Träger für Katalysatoren.

Des Weiteren wird die Herstellung von hyperverzweigten Polymeren in J. Chem. Soc. Perkin Trans. 1992 (Seiten 2459 - 2469) beschrieben, wobei das hyperverzweigte Polymer in einem Mehrschrittverfahren hergestellt wird.

In Macromolecules 1993, 26, Seiten 4617 - 4623 wird die Herstellung von einem aromatischen hyperverzweigten Polyester mit Phenol und Acetat Endgruppen dargelegt. Die Synthese basiert auf einer Schmelzkondensation von A₂B Monomeren, beispielsweise 3,5(Bis-trimethylsiloxy)benzoylchlorid und 3,5-Diacetoxybenzoesäure.

US 5,041,516 offenbart ein Mehrschrittverfahren zur Herstellung von Polyestern aus aliphatischen und aromatischen Monomeren.

Darüber hinaus wird die Herstellung von Polyestern aus aromatischen und aliphatischen Monomeren in US 5,136,014 offenbart. Des Weiteren beschreibt die Patentschrift US 5,183,862 die Herstellung von hyperverzweigten Polyestern aus aliphatischen und aromatischen Monomeren.

Des Weiteren legt die Schrift US 5,196,502 die Darstellung von hyperverzweigten aromatischen Polyestern unter Verwendung von Diacetoxybenzoesäuren und Monoacetoxydibenzoesäuren dar.

Die Schrift US 5,225,522 offenbart mehrfach verzweigte Polyester mit aliphatischen und aromatischen Gruppen sowie Verfahren zu deren Herstellung.

Des weiteren beschreibt die Druckschrift US 5,227,462 hyperverzweigte Poly-ester, die sowohl aliphatische als auch aromatische Gruppen umfassen können.

Zusammensetzungen zur Herstellung von Tinten, die hyperverzweigte Polymere umfassen, werden in US 5,266,106 offenbart.

Die Herstellung von Oberflächen funktionalisierten Polymerpartikeln wird in US 5,306,561 dargestellt.

Die Druckschrift US 5,362,843 offenbart ein Verfahren zur Herstellung von hyperverzweigten Makromolekülen.

Die Druckschrift US 5,418,301 offenbart ein Verfahren zur Herstellung von den dendritischen Makromolekülen.

Des Weiteren beschreibt die Druckschrift WO 98/30604 Zusammensetzungen, die hyperverzweigte Polymere sowie Metallverbindungen umfassen können. Hierbei werden insbesondere Polyamidoamine sowie Polyester dargelegt.

Des Weiteren wird in WO 2004/072153 die Verwendung von hyperverzweigten Polymeren als Trägerstoff für Arzneimittel dargelegt, wobei das Trägermolekül eine retardierte Freisetzung sowie ein Transport der Arzneistoffe in die Zellen erleichtern soll. In diesem Zusammenhang werden insbesondere modifizierte Dendrimere dargelegt, die Stickstoff umfassende Gruppen aufweisen.

Des Weiteren beschreibt die Druckschrift WO 00/065024 Polymere mit Zellen zur Verkapselung von hydrophoben Molekülen. Hierbei wird eine Vielzahl von hydrophoben Resten an ein Polyolkern gebunden, wobei das erhaltene Polymer anschließend durch Polyalkylenoxide umgesetzt wird, um ein wasserlösliches Polymer zu erhalten.

Des Weiteren beschreibt die Druckschrift WO 2005/034909 Zusammensetzungen umfassend ein hyperverzweigtes Polymer, welches an einen biologisch aktiven Rest gekoppelt ist.

Darüber hinaus beschreibt die Druckschrift WO 03/037383 Präparate, die hyperverzweigte Polymere umfassen. Als hyperverzweigte Polymere werden insbesondere Polyamidoamine oder Polypropylenamine dargelegt.

Des Weiteren werden hyperverzweigte Polymere in der Druckschrift

WO 00/06267 beschrieben, wobei als hyperverzweigte Polymere insbesondere Polyetherimide dargelegt werden.

Weiterhin werden Präparate, die dendritische Polymere und pharmazeutische Wirkstoffe umfassen, in WO 03/033027 dargelegt, wobei das Dendrimer katonische Gruppen umfasst.
a) Des Weiteren wird die Verwendung von hyperverzweigten Polymeren zur gezielten Freisetzung von Wirkstoffen von Zou et al. Macromol. Biosci. 5 (2005) 662-668 beschrieben. Hierbei werden hyperverzweigte Polymere mit ionischen Gruppen versehen. Das Molekulargewicht der verwendeten hyperverzweigten Polymere beträgt ca. 5500 g/mol, wobei keine Schmelztemperatur des hyperverzweigten Polymers angegeben ist.
b) Weiterhin werden in den Druckschriften US 6,379,683 bzw. EP 1 034 839 B1 Nanokapseln beschrieben, die hyperverzweigte Polymere aufweisen. Die hyperverzweigten Polymere weisen ein Molekulargewicht von ca. 4800 g/mol auf.

Darüber hinaus werden in WO 97/14404 Zusammensetzungen beschrieben, die Dendrimere umfassen. Allerdings dienen diese Dendrimere nicht zur Verkapselung von Substanzen. Präparate im Sinne der vorliegenden Erfindung werden in WO 97/14404 nicht beschrieben.

Zusammensetzungen, die dentritische Polyester enthalten werden in US 6,284,233 dargelegt. Allerdings dienen die dentritischen Polyester nicht zur Verkapselung von Substanzen. Präparate im Sinne der vorliegenden Erfindung werden in US 6,284,233 nicht beschrieben.

Die Verwendung von hydroxyfunktionalisierten dentritischen Polymeren in kosmetischen Zusammensetzungen wird in WO 01/17485 beschrieben. Allerdings enthalten diese Zusammensetzungen keine Präparate im Sinne der vorliegenden Erfindung.

Die Dissertation von S. Suttiruengwong "Silica Aerogels and Hyperbranched Polymers as Drug Delivery Systems", Erlangen 2005, beschreibt verkapselte Systeme,' die hyperverzweigte Polymere aufweisen können. Allerdings weisen die beschriebenen Polymere ein Molekulargewicht von höchstens 4600 g/mol auf, wobei keine Schmelztemperatur des hyperverzweigten Polymers angegeben ist.

Die Druckschrift DE 10 2004 026 904 beschreibt hyperverzweigte Polyester, die insbesondere als Bestandteil von Druckfarben zweckmäßig sind. Allerdings dienen diese hyperverzweigten Polyester nicht zur Verkapselung von Substanzen. Präparate im Sinne der vorliegenden Erfindung werden in DE 10 2004 026 904 nicht beschrieben.

Darüber hinaus beschreibt die Druckschrift US 6,525,170 hyperverzweigte Polyester. Allerdings umfassen die Polyester der Beispiele einen hydrophoben Kern. Weiterhin weisen die Polymere ein *Molekulargewicht von ca. 1400 g/mol vor verschiedenen Umsetzungen auf, wobei die Eigenschaften der nach der Modifikation erhaltenen Polymere nicht ausführlich beschrieben werden. Darüber hinaus fehlen Angaben zu Glasübergangstemperaturen oder Schmelzpunkten der erhaltenen Polymere.

Ferner offenbart die Druckschrift US 2002/0002242 hyperverzweigte Polymere, die zur Modifikation von Oberflächeneigenschaften eingesetzt werden. Hierbei kann der Kern dieser Polymere hydrophob sein, wobei die Polymere insgesamt ambiphile Eigenschaften aufweisen. Diese Polymere dienen nicht zum verkapseln von niedermolekularen Substanzen. Präparate im Sinne der vorliegenden Erfindung werden nicht beschrieben.

Die Druckschrift US 2003/0114553 beschreibt Dentalmassen, die Sternpolymere umfassen können. Allerdings werden keine hyperverzweigten Polymere mit einem hydrophilen Kern und hydrophoben Endgruppen dargelegt. Präparate zur verzögerten Freisetzung von niedermolekularen Verbindungen werden ebenfalls nicht beschrieben.

Darüber hinaus beschreibt das Dokument US 2004/016394 kosmetische Zusammensetzungen, die hyperverzweigte Polymere umfassen können. Allerdings werden die Eigenschaften dieser hyperverzweigten Polymere, insbesondere das Molekulargewicht, der Verzweigungsgrad, die Hydroxyzahl oder der Schmelzpunkt nicht explizit aufgeführt.

Weiterhin werden hyperverzweigte Polymere in WO 93/17060 dargelegt. Allerdings werden diese Polymere nicht im Zusammenhang mit der gesteuerten Freisetzung von niedermolekularen Verbindungen beschrieben. Präparate im Sinne der vorliegenden Erfindung werden nicht dargelegt.

Die Druckschriften WO 2006/031388 offenbart dentritische Polymere, die insbesondere in orthopädischen Anwendungen eingesetzt werden können. Allerdings werden sehr viele unterschiedliche Polymere dargelegt, wobei keines der explizit dargelegten Polymere sämtliche Eigenschaften der vorliegend zu verwendenden hyperverzweigten Polymere aufweist. So fehlen insbesondere Angaben zum Schmelzpunkt der Polymere. Weiterhin werden keine hyperverzweigten Polymere, mit einem hydrophilen Kern und hydrophoben Endgruppen beschrieben, die ein Molekulargewicht von mindestens 6000 g/mol aufweisen.

Die Druckschriften WO 2006/031358 offenbart dentritische Polymere, die insbesondere in ophtalmischen Anwendungen eingesetzt werden können. Allerdings werden sehr viele unterschiedliche Polymere dargelegt, wobei keines der explizit dargelegten Polymere sämtliche Eigenschaften der vorliegend zu verwendenden hyperverzweigten Polymere aufweist. So fehlen insbesondere Angaben zum Schmelzpunkt der Polymere. Weiterhin werden keine hyperverzweigten Polymere mit einem hydrophilen Kern und hydrophoben Endgruppen beschrieben, die ein Molekulargewicht von mindestens 6000 g/mol aufweisen. beschreiben

Dementsprechend ist festzuhalten, dass eine Vielzahl von Präparaten beschrieben ist, die hyperverzweigte Polymere sowie niedermolekulare Verbindungen umfassen. Jedoch besteht das dauerhafte Bestreben möglichst vorteilhafte Präparate zur Verfügung zu stellen.

In Anbetracht des hierin angegebenen und diskutierten Standes der Technik war es mit die Aufgabe der vorliegenden Erfindung Präparate zur Verfügung zu stellen, die ein hervorragendes Eigenschaftsprofil aufweisen.

Insbesondere sollten die erfindungsgemäßen Präparate die niedermolekulare Substanz in einem gewählten Medium möglichst über einen langen Zeitraum freisetzen, wobei die Freisetzung möglichst konstant sein sollte.

Gemäß einem weiteren Aspekt sollte die Freisetzung der niedermolekularen Substanz möglichst einfach und zuverlässig gesteuert werden können.

Weiterhin war es Aufgabe der vorliegenden Erfindung Präparate zur Verfügung zu stellen, die einen besonders hohen Gehalt an niedermolekularer Substanz umfassen.

Des Weiteren sollte das Präparat eine besonders hohe Stabilität zeigen, wodurch insbesondere empfindliche niedermolekulare Substanzen über einen besonders langen Zeitraum gelagert werden können, ohne dass die Eigenschaften der niedermolekularen Substanz wesentlich geändert werden. Hierbei sollten die Präparate ebenfalls eine hohe Scherstabilität aufweisen, so dass eine einfache und problemlose Verarbeitung der Präparate möglich ist.

Gelöst werden diese Aufgaben sowie weitere, die zwar nicht explizit genannt werden, sich aber aus den hierin diskutierten Zusammenhängen wie selbstverständlich ableiten lassen oder sich aus diesen zwangsläufig ergeben, durch die in Anspruch 1 beschriebenen Präparate. Zweckmäßige Abwandlungen dieser Präparate werden in den auf Anspruch 1 rückbezogenen Unteransprüchen unter Schutz gestellt.

Dadurch, dass ein hyperverzweigtes Polymer einen hydrophilen Kern mit Polyestereinheiten sowie hydrophobe Endgruppen umfasst,
wobei das hyperverzweigte Polymer ein Molekulargewicht größer oder gleich 6000 g/mol und eine Hydroxyzahl im Bereich von 0 bis 200 mg KOH/g aufweist, der Verzweigungsgrad im Bereich von 20 bis 70% liegt und das hyperverzweigte Polymer eine Schmelztemperatur von mindestens 30°C aufweist, gelingt es auf nicht ohne weiteres vorhersehbarer Weise, Präparate umfassend mindestens eine niedermolekulare Substanz und mindestens ein hyperverzweigtes Polymer zur Verfügung zu stellen, die ein verbessertes Eigenschaftsprofil aufweisen.

Durch die erfindungsgemäßen Maßnahmen können ein oder mehrere der folgenden Vorteile erzielt werden:
Gemäß einem besonderen Aspekt setzen die erfindungsgemäßen Präparate die niedermolekulare Substanz in einer Vielzahl von Medien über einen besonders langen Zeitraum frei.
Gemäß einer weiteren Ausführungsform der vorliegenden Erfindung kann die Freisetzung der niedermolekularen Substanz nach einer langen Lagerzeit innerhalb einer kurzen Dauer erfolgen.

In beiden Ausführungsformen kann die Freisetzung durch externe Parameter, beispielsweise Temperatur, pH, Strahlungsfrequenz und Art des Präparat-umgebenden Mediums gezielt gesteuert werden.

Weiterhin ermöglicht das vorliegend dargelegte Präparat eine besonders stabile Aufbewahrung von empfindlichen niedermolekularen Substanzen. Dementsprechend können die Substanzen sicher und einfach gelagert werden, wobei vielfach eine Feuer- und/oder Explosionsgefahr vermindert werden kann.

Darüber hinaus können chemisch aktive, insbesondere oxidative Substanzen aufbewahrt werden, ohne dass diese Substanzen in dem Medium aktiv werden.

Darüber hinaus sind die erfindungsgemäßen Präparate überraschend stabil, so dass diese über einen langen Zeitraum aufbewahrt werden können, ohne dass ein Abbau erfolgt. Des Weiteren können die Präparate aufgrund der hohen Scherstabilität einfach und kostengünstig verarbeitet werden.

Des Weiteren sind, je nach niedermolekularer Substanz, die erfindungsgemäßen Präparate gesundheitlich unbedenklich. Weiterhin können die erfindungsgemäßen Präparate einen besonders hohen Anteil an niedermolekularer Substanz umfassen.

Aufgrund der hochverzweigten Polymerstruktur und der Möglichkeit, die Löslichkeitseigenschaften des Polymers über die Anzahl an hydrophoben Gruppen einzustellen, kann gegenüber dem Stand der Technik eine bessere Kontrolle über die Menge an niedermolekularer Substanz erzielt werden, die pro Zeiteinheit freigesetzt wird.

Die Beladungskonzentration im erfindungsgemäßen Präparat an niedermolekularer Substanz pro g Trägerpolymer kann aufgrund der hyperverzweigten Polymerstruktur und der Möglichkeit, die Löslichkeitseigenschaften des Polymers über die Anzahl an hydrophoben Gruppen einzustellen, anwendungsspezifisch eingestellt werden. Hierbei können auch Beladungskonzentrationen erreicht werden, die deutlich über den Beladungskonzentrationen des Stands der Technik (über 20 Massenprozent an niedermolekularer Substanz bezogen auf das binäre System bestehend aus Polymer und niedermolekularer Substanz) liegen.

Überraschenderweise wurde gefunden, dass bei hyperverzweigten Trägerpolymeren die Verkapselungsverfahren mit deutlich reduzierten Mengen an Lösungsmitteln bzw. komprimierten Gasen betrieben werden können. Das hyperverzweigte Polymer kann somit selbst als Lösungsmittel/Dispergiermittel fungieren. Die hierdurch reduzierten Lösungsmittel-/Gaskonzentrationen führen im Vergleich zum Stand der Technik zu sichereren Prozessen, da hyperverzweigte Polymere keine explosionsfähigen Dämpfe wie andere Lösungsmittel des Stands der Technik bilden können.

Die erfindungsgemäßen Präparate umfassen ein hyperverzweigtes Polymer mit einem hydrophilen Kern. Hydrophil bedeutet, dass der Kern in der Lage ist, einen hohen Anteil an Wasser aufzunehmen. Gemäß einem bevorzugten Aspekt der vorliegenden Erfindung ist der hydrophile Kern in Wasser löslich. Vorzugsweise beträgt die Löslichkeit in Wasser bei 90°C mindestens 10 Massenprozent, besonders bevorzugt mindestens 20 Massenprozent. Diese Größe wird anhand des hyperverzweigten Polymers vor der Hydrophobisierung, d.h. am hydrophilen Kern als solchen, gemessen. Die Messung kann gemäß der sogenannten Kolbenmethode erfolgen, wobei die Wasserlöslichkeit der reinen Substanz gemessen wird.

Bei dieser Methode wird die Substanz (Feststoffe müssen pulverisiert werden) bei einer Temperatur in Wasser aufgelöst, die leicht über der Prüftemperatur liegt. Wenn die Sättigung erreicht ist, wird die Lösung abgekühlt und bei der Prüftemperatur gehalten. Die Lösung wird gerührt, bis das Gleichgewicht erreicht ist. Alternativ kann die Messung unmittelbar bei der Prüftemperatur durchgeführt werden, wenn durch entsprechende Probenahme gesichert ist, dass das Sättigungsgleichgewicht erreicht ist. Dann wird die Konzentration der Prüfsubstanz in der wässrigen Lösung, die keine ungelösten Substanzteilchen enthalten darf, mit einer geeigneten Analysenmethode bestimmt.

Der hydrophile Kern weist vorzugsweise eine vor der Hydrophobisierung gemessene Hydroxyzahl im Bereich von 400 bis 600 mg KOH/g, besonders bevorzugt im Bereich von 450 bis 550 mg KOH/g auf. Diese Eigenschaft wird gemäß ASTM E222 gemessen. Hierbei wird das Polymer mit einer definierten Menge an Essigsäureanhydrid umgesetzt. Nicht umgesetztes Essigsäureanhydrid wird mit Wasser hydrolysiert. Anschließend wird die Mischung mit NaOH titriert. Die Hydroxyzahl ergibt sich aus dem Unterschied zwischen einer Vergleichsprobe und dem für das Polymer gemessenen Wert. Hierbei ist die Anzahl an Säuregruppen des Polymers zu berücksichtigen.

Das erfindungsgemäße hyperverzweigte Polymer weist einen Kern auf, der Polyestereinheiten umfasst. Hyperverzweigte Polymere mit Polyestereinheiten sind insbesondere in EP 0 630 389 dargelegt. Im Allgemeinen ist meist der hydrophile Kern eine zentrale Einheit auf die von einem Initiatormolekül mit mindestens 2, vorzugsweise mindestens 3 Hydroxygruppen abgeleitet ist, und Wiederholungseinheiten auf die von Monomeren mit mindestens einer Carbonylgruppe und mindestens 2 Hydroxygruppen abgeleitet sind.

Hochverzweigte, globulare Polymere werden in der Fachliteratur auch als "dendritische Polymere" bezeichnet. Diese aus multifunktionellen Monomeren synthetisierten dendritischen Polymere lassen sich in zwei unterschiedliche Kategorien einteilen, die "Dendrimere" sowie die "hyperverzweigten Polymere". Dendrimere besitzen einen sehr regelmäßigen, radialsymmetrischen Generationenaufbau. Sie stellen monodisperse globulare Polymere da, die im Vergleich zu hyperverzweigten Polymeren - in Vielschrittsynthesen mit einem hohen Syntheseaufwand hergestellt werden. Dabei ist die Struktur durch drei unterschiedliche Areale charakterisiert: - dem polyfunktionellen Kern, der das Symmetriezentrum darstellt, - verschiedenen wohldefinierten radialsymmetrischen Schichten einer Wiederholungseinheit (Generation) und - den terminalen Gruppen. Die hyperverzweigten Polymere sind im Gegensatz zu den Dendrimeren polydispers und hinsichtlich ihrer Verzweigung und Struktur unregelmäßig. Neben den dendritischen und linearen Einheiten treten - im Gegensatz zu Dendrimeren - in hyperverzweigten Polymeren auch lineare Einheiten auf.

Bezüglich der unterschiedlichen Möglichkeiten zur Synthese von Dendrimeren und hyperverzweigten Polymeren sei auf
a) Fréchet J.M.J., Tomalia D.A., Dendrimers and Other Dendritic Polymers, John Wiley &Sons, Ltd., West Sussex, UK 2001 sowie
b) Jikei M., Kakimoto M., Hyperbranched polymers: a promising new class of materials, Prog. Polym. Sci., 26 (2001) 1233-1285 und/oder
c) Gao C., Yan D., Hyperbranched Polymers: from synthesis to applications, Prog. Polym. Sci., 29 (2004) 183-275
   verwiesen, die hiermit als Referenzen eingeführt werden und als Teil der Offenbarung der vorliegenden Erfindung gelten.

Die in diesen Druckschriften beschriebenen hyperverzweigten und hochverzweigten Polymere sind auch im Sinne der vorliegenden Erfindung bevorzugte Trägerpolymere. In diesem Zusammenhang ist es bevorzugt, dass die hyperverzweigten Polymere mindestens 3 Wiederholungseinheiten pro Molekül, vorzugsweise mindestens 10 Wiederholungseinheiten pro Molekül, ferner bevorzugt mindestens 100 Wiederholungseinheiten pro Molekül, zudem bevorzugt mindestens 200 Wiederholungseinheiten und darüber hinaus bevorzugt mindestens 400 Wiederholungseinheiten besitzen, die jeweils mindestens drei, bevorzugt mindestens vier Bindungsmöglichkeiten aufweisen, wobei mindestens 3 dieser Wiederholungseinheiten, besonders bevorzugt mindestens 10 und darüber hinaus bevorzugt mindestens 20 jeweils über mindestens drei, bevorzugt über mindestens vier Bindungsmöglichkeiten mit mindestens drei, bevorzugt mindestens vier weiteren Wiederholungseinheiten verknüpft sind.

Verschiedentlich weisen die hyperverzweigten Polymere maximal 10000, vorzugsweise maximal 5000 und besonders bevorzugt maximal 2500 Weiderholungseinheiten auf.

In einer bevorzugten Ausführungsform weist das hochverzweigte Polymer mindestens drei Wiederholungseinheiten auf, welche jeweils mindestens drei mögliche Bindungsmöglichkeiten aufweisen, wobei mindestens drei dieser Wiederholungseinheiten mindestens zwei mögliche Bindungsmöglichkeiten aufweisen. Dabei wird unter dem Begriff "Wiederholungseinheit" vorzugsweise eine stets wiederkehrende Struktur innerhalb des hyperverzweigten Moleküls verstanden. Unter dem Begriff "Bindungsmöglichkeit" wird vorzugsweise diejenige funktionelle Struktur innerhalb einer Wiederholungseinheit verstanden, mit der eine Verknüpfung zu einer anderen Wiederholungseinheit möglich ist. Bezogen auf die vorstehend dargestellten Beispiele eines Dendrimers bzw. eines hyperverzweigten Polymers ist die Wiederholungseinheit eine Struktur mit jeweils drei Bindungsmöglichkeiten (X,Y,Z):

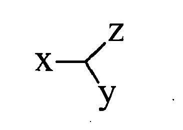

Die Verknüpfung der einzelnen Bindungseinheiten miteinander kann durch Kondensationspolymerisation, durch radikalische Polymerisation, durch anionische Polymerisation, durch kationische Polymerisation, durch Gruppentransferpolymerisation, durch koordinative Polymerisation oder durch Ringöffnungspolymerisation erfolgen.

Die Begriffe Initiatormolekül sowie Wiederholungseinheit sind in der Fachwelt weithin bekannt. So können die erfindungsgemäß einzusetzenden hyperverzweigten Polymere durch Polykondensation erhalten werden, wobei ausgehend von einem mehrwertigen Alkohol zunächst die Carbonsäuregruppen der Monomere umgesetzt werden. Hierbei werden Estergruppen gebildet. Da die Monomere mindestens 2 Hydroxygruppen umfassen, weist das Makromolekül nach jeder Umsetzung mehr Hydroxygruppen auf, als vor der Umsetzung.

Vorzugsweise ist das Initiatormolekül ein aliphatisches Polyol, mit vorzugsweise 3, 4, 5, 6, 7 oder 8, besonders bevorzugt 3, 4 oder 5 Hydroxygruppen.

Besonders bevorzugt ist das Initiatormolekül ausgewählt aus Ditrimethylolpropan, Ditrimethylolethan, Dipentaerythrit, Pentaerythrit, alkoxliertem Pentaerythrit, Trimethylolethan, Trimethylolpropan, alkoxyliertem Trimethylolpropan, Glycerin, Neopentylalkohol, Dimethylolpropan und/oder 1,3-Dioxan-5,5-dimethanol.

Gemäß einem besonderen Aspekt der vorliegenden Erfindung sind die Wiederholungseinheiten von Monomeren mit einer Carboxylgruppe und mindestens 2 Hydroxygruppen abgeleitet. Zu diesen bevorzugten Monomeren gehören insbesondere Dimethylpropionsäure, α,α-Bis(hydroxymethyl)buttersäure, α,α,α-Tris(hydroxymethyl)essigsäure, α,α-Bis(hydroxymethyl)valariansäure, α,α-Bis(hydroxy)propionsäure und/oder 3,5-Dihydroxybenzoesäure.

Ganz besonders.bevorzugt ist der hydrophile Kern durch Polymerisation von Dimethylolpropionsäure erhältlich, wobei als Initiatormolekül besonders bevorzugt Ditrimethylolpropan, Trimethylolpropan, ethoxyliertes Pentaerthrit, Pentaerthrit oder Glycerin eingesetzt wird.

Gemäß einer bevorzugten Ausführungsform weist der hydrophile Kern vorzugsweise ein Molekulargewicht von mindestens 1500 g/mol, bevorzugt mindestens 2500 g/mol. Diese Größe bezieht sich auf das Gewichtsmittel des Molekulargewichts (Mw), welches mittels Gelpermationschromatographie gemessen werden kann, wobei die Messung in DMF erfolgt und als Referenz Polyethylenglykole eingesetzt werden (vgl. u.a. Burgath et. al in Macromol.Chem. Phys., 201 (2000) 782-791). Hierbei wird eine Kalibierkurve eingesetzt, die unter Verwendung von Polystyrol-Standards erhalten wurde. Diese Größe stellt daher ein apparenter Meßwert dar.

Vorzugsweise kann der hydrophile Kern eine Glasübergangstemperatur aufweisen, die im Bereich von -40 bis 60 °C, besonders bevorzugt 0 bis 50°C und ganz besonders bevorzugt 10 bis 40°C liegt. Die Glasübergangstemperatur kann durch DSC-Verfahren ermittelt werden, wobei eine Heizrate von 3°C/min verwendet werden kann (DMA Tan δ peak; Netzch DMA 242 3-point bending 1Hz 3°C/min).

Die Hydrophobisierung der Oberfläche wird im Allgemeinen als letzter Reaktionsschritt durch Umsetzung zumindest eines Teils der freien Hydroxygruppen mit vorzugsweise einer langkettigen Carbonsäure erhalten.

Vorzugsweise beträgt der Funktionalisierungsgrad des hyperverzweigten Kernmoleküls mit hydrophoben Endgruppen, vorzugsweise mit Fettsäure-enthaltenden Bausteinen, mindestens 30%, besonders bevorzugt mindestens 40%. Gemäß einem weiteren Aspekt der vorliegenden Erfindung liegt der Funktionalisierungsgrad des hyperverzweigten Kernmoleküls mit hydrophoben Endgruppen, vorzugsweise mit Fettsäure-enthaltenden Bausteinen, im Bereich von 30 bis 100%, bevorzugt im Bereich von 35 bis 95%, insbesondere bevorzugt im Bereich von 40 bis 90% und ganz besonders bevorzugt im Bereich von 45 bis 85%.

Der Funktionalisierungsgrad bezieht sich auf den Anteil an Hydroxygruppen die bei der Hydrophobisierung umgesetzt werden. Dementsprechend kann der Funktionalisierungsgrad bzw. der Veresterungsgrad mit Fettsäuren über die Messung der Hydroxy-Zahl für das hyperverzweigte Kernmolekül vor der Hydrophobisierungsreaktion und nach der Hydrophobierungsreaktion bestimmt werden.

Neben dem hydrophilen Kern weist das hyperverzweigte Polymer hydrophobe Endgruppen auf. In diesem Zusammenhang bedeutet der Begriff hydrophobe Endgruppen, dass mindestens ein Teil der Kettenenden des hyperverzweigten Polymers hydrophobe Gruppen aufweist. Hierbei kann angenommen werden, dass hierdurch eine zumindest teilweise hydrophobisierte Oberfläche erhalten wird.

Der Begriff hydrophob ist an sich in der Fachwelt bekannt, wobei die Gruppen, die zumindest an einem Teil der Enden der hyperverzweigten Polymere vorhanden sind, für sich betrachtet, eine geringe Wasserlöslichkeit aufweisen.

Gemäß einem besonderen Aspekt wird die Oberfläche durch Gruppen hydrophobisiert, die von Carbonsäuren mit mindestens 6, bevorzugt mindestens 12 Kohlenstoffatomen abgeleitet sind. Die Carbonsäuren weisen vorzugsweise höchstens 40, besonders höchstens 32 Kohlenstoffatome, besonders bevorzugt höchstens 20 Kohlenstoffatome und ganz besonders bevorzugt höchstens 18 Kohlenstoffatome auf. Hierbei können die Gruppen von gesättigten und/oder ungesättigten Fettsäuren abgeleitet sein. Vorzugsweise beträgt der Anteil der Carbonsäuren mit 12 bis 18 Kohlenstoffatomen mindestens 30 Gew.-%, besonders bevorzugt mindestens 50 Gew.-% und ganz besonders bevorzugt mindestens 60 Gew.-%, bezogen auf das Gewicht der zur Hydrophobisierung eingesetzten Carbonsäuren.

Hierzu gehören insbesondere Fettsäuren, die in Leinsamen, Sojabohnen und/oder Tallöl enthalten sind. Besonders geeignet sind Fettsäuren, die einen geringen Anteil an Doppelbindungen aufweisen, beispielsweise Hexadecensäure, insbesondere Palmitoleinsäure, und Octadecensäure, insbesondere Ölsäure.

Bevorzugte Carbonsäuren weisen hierbei einen Schmelzpunkt von mindestens 35 °C, bevorzugt mindestens 40 °C und besonders bevorzugt mindestens 60 °C auf. Dementsprechend werden bevorzugt lineare, gesättigte Carbonsäuren eingesetzt. Hierzu gehören insbesondere Octansäure, Decansäure, Dodecansäure, Tetradecansäure, Hexadecansäure, Heptadecansäure, Octadecansäure, Eicosansäure, Docosansäure und Tetracosansäure. Besonders bevorzugt sind gesättigte Fettsäuren mit 16 bis 22 Kohlenstoffatomen, insbesondere bevorzugt 16 bis 18 Kohlenstoffatomen.

Das hyperverzweigte Trägerpolymer (nach der Hydrophobisierung) weist ein Molekulargewicht von mindestens 6000 g/mol, besonders bevorzugt mindestens 7500 g/mol auf. Vorzugsweise beträgt das Molekulargewicht höchstens 30000 g/mol, besonders bevorzugt höchstens 25000 g/mol. Diese Größe bezieht sich auf das Gewichtsmittel des Molekulargewichts (Mw), welches mittels Gelpermationschromatographie gemessen werden kann, wobei die Messung in DMF erfolgt und als Referenz Polyethylenglykole eingesetzt werden (vgl. u.a. Burgath et. al in Macromol.Chem. Phys., 201 (2000) 782-791). Hierbei wird eine Kalibierkurve eingesetzt, die unter Verwendung von Polystyrol-Standards erhalten wurde. Diese Größe stellt daher ein apparenter Meßwert dar.

Die Polydispersität Mw/Mn bevorzugter hyperverzweigter Polymere liegt vorzugsweise im Bereich von 1,01 bis 6,0, besonders bevorzugt im Bereich von 1,10 bis 5,0 und ganz besonders bevorzugt im Bereich von 1,2 bis 3,0, wobei das Zahlenmittel des Molekulargewichts (Mn) ebenfalls durch GPC erhalten werden kann.

Das Gewichtsverhältnis von hydrophilem Kern zu den hydrophoben Endgruppen kann vorzugsweise im Bereich von 10:1 bis 1:10, besonders bevorzugt 1:1 bis 1:2,5 liegen. Dieses Verhältnis ergibt sich aus dem Gewichtsmittel des hydorphilen Kerns und dem Gewichtsmittel des hyperverzweigten Polymers.

Die Viskosität des hyperverzweigten Polymeren liegt vorzugsweise im Bereich von 50 mPas bis 5,00 Pas, besonders bevorzugt im Bereich von 70 mPas bis 3,00 Pas, wobei diese Größe mittels Rotationsviskosimetrie bei 110°C und 30 s⁻¹ zwischen zwei 20 mm Platten gemessen werden kann.

Die Säurezahl des hyperverzweigten Polymers liegt vorzugsweise im Bereich von 0 bis 20 mg KOH/g, besonders bevorzugt im Bereich von 1 bis 15 mg KOH/g und ganz besonders bevorzugt im Bereich von 6 bis 10 mg KOH/g. Diese Eigenschaft kann durch Titration,mit NaOH gemessen werden (vgl. DIN 53402).

Des weiteren weist das hyperverzweigte Polymer nach der Hydrophobisierung eine Hydroxyzahl im Bereich von 0 bis 200 mg KOH/g, bevorzugt im Bereich von 1 bis 150 mg KOH/g und ganz besonders bevorzugt im Bereich von 10 bis 140 mg KOH/g auf. Diese Eigenschaft wird gemäß ASTM E222 gemessen. Hierbei wird das Polymer mit einer definierten Menge an Essigsäureanhydrid umgesetzt. Nicht umgesetztes Essigsäureanhydrid wird mit Wasser hydrolysiert. Anschließend wird die Mischung mit NaOH titriert. Die Hydroxyzahl ergibt sich aus dem Unterschied zwischen einer Vergleichsprobe und dem für das Polymer gemessenen Wert. Hierbei ist die Anzahl an Säuregruppen des Polymers zu berücksichtigen. Dies kann durch die Säurezahl erfolgen, die über das zuvor beschriebenen Verfahren bestimmt werden kann.
c) Der Verzweigungsgrad des hyperverzweigten Polymers liegt im Bereich von 20 bis 70%, vorzugsweise 25 bis 60%. Der Verzweigungsgrad ist abhängig von den zur Herstellung des Polymers, insbesondere des hydrophilen Kerns eingesetzten Komponenten sowie der Reaktionsbedingungen. Der Verzweigungsgrad kann gemäß Frey et al. bestimmt werden, wobei dieses Verfahren in D.Hölter, A.Burgath, H.Frey, Acta Polymer, 1997, 48, 30 und H. Magnusson,E. Malmström, A. Hult, M. Joansson, Polymer 2002, 43, 301 dargelegt ist.

Das hyperverzweigte Polymer weist eine Schmelztemperatur von mindestens 30 °C, besonders bevorzugt mindestens 35 °C und ganz besonders bevorzugt mindestens 40 °C auf. Gemäß einem besonderen Aspekt der vorliegenden Erfindung kann der Schmelzpunkt des hyperverzweigten Polymers vorzugsweise höchstens 65°C, insbesondere bevorzugt höchstens 60°C, besonders bevorzugt höchstens' 57°C und ganz besonders bevorzugt höchstens 55°C betragen. Die Schmelztemperatur kann mittels Differential Scanning Calometry (DSC) erfolgen, z.B. mit dem Apparat Mettler DSC 27 HP und einer Heizrate von 10°C/min.

Die Wasserlöslichkeit des hyperverzweigten Polymers nach der Hydrophobisierung beträgt vorzugsweise höchstens 10 Massenprozent, besonders bevorzugt höchstens 7 Massenprozent und ganz besonders bevorzugt höchstens 5 Massenprozent, gemessen nach der zuvor dargelegten Kolbenmethode bei 40°C.

Des Weiteren zeigen besonders bevorzugte hyperverzweigte Polymere beim Mischen mit Wasser bei einer Temperatur von 60°C oder weniger, vorzugsweise von 50 °C oder weniger und besonders bevorzugt ca. 48°C lediglich Flüssig-Flüssig-Gleichgewichte, jedoch keine Fest-Flüssig-Gleichgewichte oder Fest-Flüssig-Flüssig-Gleichgewichte, wobei zur Bestimmung dieser Eigenschaft eine Mischung von 50 Gew.-% Wasser und 50 Gew.-% hyperverzweigtes Polymer verwendet wird. Die Messung kann über Filterexperimente erfolgen, wobei die Mischung durch ein Filter mit einer geeigneten Porengröße, die vorzugsweise 20 µm oder weniger beträgt; filtriert wird. Bei Vorhandensein von Fest-Flüssig-Gleichgewichten oder Fest-Flüssig-Flüssig-Gleichgewichten bleiben die festen Bestandteile der Gleichgewichte im Filter zurück, wohingegen bei Flüssig-Flüssig-Gleichgewichten kein Rückstand beobachtet wird. Diese Eigenschaft kann unter anderem durch den Funktionalisierungsgrad sowie die zur Hydrophobisierung eingesetzten Carbonsäuren gesteuert werden. Weisen die hyperverzweigten Polymere einen geringen Funktionalisierungsgrad und/oder kurzkettige Carbonsäuren auf, so zeigen diese Polymere bei den zuvor dargelegten Bedingungen im Allgemeinen lediglich Flüssig-Flüssig-Gleichgewichte, jedoch keine Fest-Flüssig-Gleichgewichte.

Vorzugsweise besteht das hyperverzweigte Polymer im Wesentlichen aus Wasserstoff, Sauerstoff und Kohlenstoff. Der Begriff im Wesentlichen bedeutet, dass weitere Elemente bis zu höchstens 10 Gew. %, besonders bevorzugt höchstens 5 Gew. % in dem hyperverzweigten Polymer enthalten sind.

Gemäß einem besonderen Aspekt der vorliegenden Erfindung kann das hyperverzweigte Polymer enzymatisch abgebaut werden. Dies kann beispielsweise dadurch erreicht werden, dass der hydrophile Kern und/oder die hydrophobe Hülle enzymatisch abbaubare organische Estergruppen umfasst. Bevorzugte hyperverzweigte Polymere oder Präparate gemäß der vorliegenden Erfindung zeigen einen enzymatischen Abbau von drei Tagen oder weniger, bevorzugt 2 Tagen oder weniger und besonders bevorzugt von einem Tag oder weniger. Hierbei werden Präparate mit einem geeigneten Enzym, insbesondere einer Lipase, beispielsweise Lipomod 34P (Biocatalyst Lmt., UK) abgebaut, wobei die Zeit gemessen wird bis zu der 50 Gew.-% des Wirkstoffs freigesetzt wurden. Beispielsweise können Präparate mit einem Beladungsgrad von 10 bis 20 Gew.-% untersucht werden, wobei vorzugsweise 0,22 g mit Wirkstoff-beladene Polymerpartikel in 15 ml Phosphatpuffer (pH 5.01) oder in 15 ml Lösung des Enzyms Lipomod 34P (Biocatalyst Lmt., UK) in dem gleichen Puffer (Konzentration des Lipomods 34P betrug 0.5 mg/ml) suspendiert werden können. Die Proben können in einem Wasserbad bei 37°C ohne Durchmischung gehalten werden. In regelmäßigen Zeitabständen, beispielsweise 5 Stunden, können Proben von ca. 2 ml entnommen werden, wobei die Konzentration des Wirkstoffs mit geeigneten Verfahren, beispielsweise HPLC (High Pressure Liquid Chromatography) analysiert werden kann. Hierbei wird die Freisetzung einer Vergleichsprobe, die kein Enzym umfasst, berücksichtigt, um Probleme der Lagerstabilität bei den gewählten Messbedingungen auszuschließen, so dass sich der zuvor angegebene Wert auf die Differenz zwischen gemessener Probe und Vergleichsprobe ergibt.

Die Herstellung dieser hyperverzweigten Polymere ist insbesondere in EP 630 389 dargelegt. Im Allgemeinen kann ein Initiatormolekül mit mindestens einer Verbindung umgesetzt werden, die mindestens 2 Hydroxygruppen sowie mindestens eine Carbonsäuregruppe umfasst. Hierdurch wird ein hydrophiler Kern erhalten, der mit mindestens einer hydrophoben Verbindung, beispielsweise einer langkettigen Carbonsäure umgesetzt werden kann.

Im Allgemeinen wird die Reaktion bei einer Temperatur im Bereich von 0°C bis 300°C, vorzugsweise 100°C bis 250°C durchgeführt, wobei die Umsetzung durch bekannte Veresterungskatalysatoren beschleunigt werden kann. Zu diesen Katalysatoren gehören beispielsweise Lewis- und Bronstedtsäuren, insbesondere p-Toluolsulfonsäure, Methansulfonsäure, Trifluoressigsäure, BF₃, AlCl₃ und SnCl₄; Titanverbindungen, insbesondere Tetrabutyltitanat; Zink- und/oder Zinnpulver.

Vorzugsweise wird bei der Veresterung freigesetztes Wasser aus der Reaktionsmischung entfernt.

Neben einem hyperverzweigten Polymer umfassen die erfindungsgemäßen Präparate mindestens eine niedermolekulare Substanz. Hierbei wird die niedermolekulare Substanz vorzugsweise durch eine nicht kovalente Weise mit dem hyperverzweigten Polymer verbunden. Dies kann beispielsweise durch ionische oder polare Wechselwirkungen oder durch Van-der-Waals Kräfte erfolgen.

Aufgrund der Wechselwirkung von hyperverzweigtem Polymer und niedermolekularer Substanz kann sich das Präparat der vorliegenden Erfindung von einer konventionellen Mischung dieser Komponenten unterscheiden.

Diese Wechselwirkung kann auf bekannte Weise gemessen werden. Je nach niedermolekularer Substanz sind hierfür vielfach spektroskopische Methoden geeignet. Beispielsweise können teilweise Verschiebungen im Infrarotspektrum beobachtet werden.

Des Weiteren können die erfindungsgemäßen Präparate gegenüber einer konventionellen Mischung eine verzögerte Freisetzung der niedermolekularen Substanz in ein Medium zeigen, welches von der niedermolekularen Substanz des Präparats verschieden ist. Die verzögerte Freisetzung kann gemäß der von Smirnova, I.; Suttiruengwong, S.; Arlt, W. "Feasibility study of hydrophilic and hydrophobic silica aerogels as drug delivery systems"; Journal of Non-Crystalline Solids (2004) 54-60, beschriebenen Verfahren gemessen werden.

Im Allgemeinen beträgt der Zeitunterschied, um eine identische Konzentration der niedermolekularen Substanz in dem Medium zu erhalten, in das die niedermolekulare Substanz freigesetzt wird, mindestens 1 Minute, bevorzugt mindestens 5 Minuten. Hierbei bezieht sich dieser Zeitunterschied auf die Messung von einem Präparat der vorliegenden Erfindung und die Messung einer konventionellen Mischung dieser Komponenten unter identischen Bedingungen einer verzögerten Freisetzung. Verzögerte Freisetzung bedeutet, dass die Bedingungen nicht so gewählt werden, dass das Präparat die niedermolekulare Substanz möglichst schnell freisetzt. Diese Bedingungen sind dem Fachmann bei Kenntnis dieser Anmeldung geläufig. Die Werte der konventionellen Mischung können auch durch getrennte Zugabe der Komponenten ermittelt werden.

Nach einer besonderen -Ausführungsform liegt das Präparat verkapselt vor, wobei der Begriff "Verkapselung" in der Fachwelt bekannt ist. Im verkapselten Präparat kann beispielsweise die niedermolekulare Substanz in einer Hülle eingebettet werden, die hyperverzweigtes Polymer umfasst. Dies kann beispielsweise durch eine Matrixverkapselung und/oder eine Kern-Hülle-Verkapselung erfolgen.

Gemäß einem besonderen Aspekt der vorliegenden Erfindung kann das Präparat der vorliegenden Erfindung partikelförmig vorliegen. Hierbei weisen diese Partikel vorzugsweise eine Größe im Bereich von 1 bis 1000 µm, besonders bevorzugt 10 bis 500 µm auf.

Die Form der Partikel ist an sich unkritisch, wobei die Partikel jedoch vorzugsweise eine sphärische Form aufweisen.

Der Begriff sphärisch bezeichnet im Rahmen der vorliegenden Erfindung, dass die Partikel vorzugsweise eine kugelförmige Gestalt aufweisen, wobei dem Fachmann offensichtlich ist, dass aufgrund der Herstellungsmethoden auch Partikel mit anderer Gestalt enthalten sein können, oder dass die Form der Partikel von der idealen Kugelgestalt abweichen kann.

Dementsprechend bedeutet der Begriff sphärisch, dass das Verhältnis von der größten Ausdehnung der Partikel zur geringsten Ausdehnung maximal 4, vorzugsweise maximal 2 beträgt, wobei diese Ausdehnungen jeweils durch den Schwerpunkt der Partikel gemessen werden. Vorzugsweise sind mindestens 70%, besonders bevorzugt mindestens 90 %, bezogen auf die Zahl der Partikel, sphärisch.

Die Partikelgröße kann auf allgemein bekannte Weise bestimmt werden. Hierzu können beispielsweise mikroskopische Aufnahmen verwendet werden, die visuell und/oder mit Hilfe von Computern ausgewertet werden können.

Des Weiteren weisen bevorzugte Mikropartikel eine besonders enge Partikelgrößenverteilung auf. So liegen bevorzugt mindestens 80 Gew.-% der Partikel innerhalb eines Größenbereichs von höchstens 200 µm, vorzugsweise höchstens 100 µm, besonders bevorzugt höchstens 50 µm.

Gemäß einem besonderen Aspekt der vorliegenden Erfindung weisen vorzugsweise 90% der Partikel eine Größe im Bereich von 1 bis 1000 µm, insbesondere bevorzugt 3 bis 800 µm, besonders bevorzugte 7 bis 700 µm und ganz besonders bevorzugt 10 bis 500 µm auf.

Die Präparate der vorliegenden Erfindung können eine hervorragende Scherstabilität zeigen, die vielfach über die Wahl des hyperverzweigten Polymers und die Verfahrensbedingungen bei der Präparatherstellüng beeinflusst werden kann. Vorzugsweise zeigen die Präparate eine Scherstabilität von 1 Minute oder länger, bevorzugt 5 Minuten oder länger, wobei diese Stabilität bei einer Belastung bestimmt wird, die der eines ULTRA-TURRAX Rührers bei 15000 Umdrehungen pro Minute, bevorzugt 20000 Umdrehungen pro Minute entspricht. Im Allgemeinen wird hierbei eine Dispersion, beispielsweise in einem Pharmaöl (Paraffin Oil WINOG 100 Pharma von Univar GmbH) hergestellt, wobei die Dispersion beispielsweise 10 Gew.-% Präparat enthalten kann. Die Partikel werden vor und nach der Stabilitätsmessung, die beispielsweise durch einen ULTRA-TURRAX Rührers bei 15000 Umdrehungen pro Minute, bevorzugt 20000 Umdrehungen pro Minute erfolgen kann, mikroskopisch untersucht, wobei die Partikelform, Größe und Verteilung beurteilt wird. Eine Scherstabilität bei den zuvor genannten Bedingungen ist gegeben, falls keine wesentlichen Veränderungen beobachtet werden können.

Die Lagerstabilität der erfindungsgemäßen Präparate ist vielfach ebenfalls überraschend hoch, wobei diese von der Art und Zusammensetzung des Medium, falls die Präparate in Form von Dispersionen oder Emulsionen gelagert werden, und/oder den Lagertemperaturen abhängig ist. Bei bevorzugten Lagerbedingungen können bevorzugte Präparate 'über einen langen Zeitraum, beispielsweise 10 Tage oder länger, vorzugsweise 30 Tage oder länger und besonders bevorzugt 90 Tage oder länger gelagert werden. Diese Größe kann durch die Freisetzung von Wirkstoff in ein Medium oder den Abbau der niedermolekularen Substanz gemessen werden. Hierbei beziehen sich diese Angaben auf den Zeitraum bis zu dem höchstens 10 % der niedermolekularen Substanz in ein Medium freigesetzt wurden, in dem das Präparat gelagert werden kann bzw. die Zeit bis zu der höchstens 10 % der niedermolekularen Substanz, beispielsweise durch Oxidation abgebaut wurden.

Die niedermolekulare Substanz weist vorzugsweise eine Molmasse im Bereich von 15 g/mol bis 1000 g/mol, besonders bevorzugt 30 g/mol bis 800 g/mol und ganz besonders bevorzugt 60 g/mol bis 500 g/mol auf.

Die erfindungsgemäße molekulare Substanz kann auf einem weiten Gebiet gewählt werden. Hierzu gehören insbesondere Verbindungen, die eine Peroxidgruppe umfassen, Aminosäuren, Katalysatoren, Farbstoffe und/oder Pigmente, Vitamine, Monomere, Geschmacks- und/oder Aromastoffe, biologisch aktive Komponenten, insbesondere ein Arzneimittel, Initiatoren, Persulfate, Silikone, Tenside, Kieselsäuren, Silane, Enzyme und Coenzyme, Lösungsmittel, Füllstoffe, Reaktivvernetzer, Detergentien, Haarfarben, Betonzusatzmittel oder Pflanzenextrakte.

Überraschend ermöglichen die Präparate der vorliegenden Erfindung, dass insbesondere chemisch labile Verbindungen, beispielsweise Initiatoren und/oder Persulfate sicher gelagert und transportiert werden können, wobei die Lager- und Transportbedingungen wesentlich weniger anspruchsvoll sind als die von Lösungen oder Pulver dieser Substanzen. Hierbei kann das Risiko von Explosionen oder Flammbildung unerwartet stark verringert werden.

Zu den bevorzugten biologisch aktiven Komponenten gehören insbesondere Peptide, Vitamine und Vitaminvorstufen, Fette und Fettsäuren, Aminosäuren und Aminosäurevorstufen, beispielsweise Kreatin, Zucker und Zuckerderivate, Nukleotide, Nukleinsäuren sowie Vorstufen und Derivate derselben, beispielsweise DNA- und RNA-Oligomere.

Zu den Vitaminen gehören insbesondere Vitamin A, Vitamine des B-Komplexes, beispielsweise Vitamin B1, Vitamin B2, Vitamin B3 (Folsäure) und Vitamin B12, Vitamin C (Ascorbinsäure), Vitamine des D-Komplexes, insbesondere 7-Dehydrocholesterol, Lumisterol, Calciferol, Ergocalciferol, Cholecalciferol, 22,23-Dihydroergocalciferol und Sitocalciferol, und Vitamin E (Tocopherol) und Vitamin K (Phyllochinon, Menachinon).

Zu den bevorzugten Aminosäuren gehören, insbesondere DL-Methionin, L-Lysin, L-Threonin, L-Tryptophan und L-Leucin.

Des Weiteren können die erfindungsgemäßen Präparate als niedermolekulare Substanz insbesondere Geschmacksstoffe, Aromastoffe, natürliche Extrakte, geschmacksverstärkende Verbindungen, naturidentische Aromastoffe sowie Enzym modifizierte Nahrungsmittelzusätze umfassen.

Zu den Aromastoffen gehören insbesondere Ketone, Aldehyde, schwefelhaltige Verbindungen, Carbonsäureester, Alkohole und/oder natürliche Extrakte.

Zu den bevorzugt einzusetzenden Ketonen gehören, beispielsweise Aceton, Acetophenon, 2,6-Dimethyl-4-Heptanon, 3-Decen-2-on, Methylamylketon, Methylethylketon, Methylheptylketon, Methylnonylketon, 4-Methyl-2-pentanon, Methylpropylketon, 1-Methyl-4-isopropenyl-6-cyclohexen-2-on (D,L-Carvon) und/oder Propiophenon.

Zu den bevorzugt einzusetzenden Aldehyden gehören insbesondere Acetaldehyde, Butylaldehyde, Zimtsäurealdehyd, Decanal, Dodecanal, Heptanal, Hexanal, Isobutyraldehyd, E-2-Decenal, E-2-Dodecanal, E-2-Hexanal, E-2-Nonenal, E-2-Octenal, 2,4 Decadienal, 2,4 Dodecadienal, 2,4 Heptadienal, 2,4 Nonadienal und/oder 2.4 Octadienal.

Zu den bevorzugt' einzusetzenden schwefelhaltigen Verbindungen gehören unter anderem
Sulfide, wie beispielsweise Dimethyldisulfid, Dimethyltrisulfid, Diphenyldisulfid, Dipropyldisulfid, Dipropyltrisulfid, Ethylmethylsulfid, Isopropyldisulfid, Methylpropyldisulfid, Methylpropyltrisulfid, Methyl-2-thiofuroat, 4-Methylthio-2-butanon, 3-Methylthio-1-hexylacetat, 4-(Methylthio)4-methyl-2-pentanon;
Thiocarbonsäuren und Thiocarbonsäurederivate, insbesondere Thioester, wie beispielsweise Ethyl-3-(methylthio)butyrat, Ethylthioacetat, Methyl-3-(methylthio)propionat, Methylthiobenzoat, Methylthiobutyrat, Methylthiohexanoat, Methylthio-isovalerat, n-Propyl-thioacetat;
Mercaptane, insbesondere Hexylmercaptan, Isoamylmercaptan, Isobutylmercaptan und/oder
Thioketone, beispielsweise Thiomenthon.

Zu den bevorzugt einzusetzenden Carbonsäureester gehören unter anderem Amylacetat, Isoamylacetat, Ethylacetat, Ethyl p-anistat, Ethylformiat, Ethylhexanoat, Ethyloctanoat, Buttersäureester, n-Hexylacetat, n-Hexylcrotonat, Hexylisovalerat, Isoamylbutyrat, Isoamylhexanoat, Isobutylbutyrat, Isobutylpropionat, Methylbenzoat, 2-Methylbutylacetat, Zimmtsäuremethylester, Methyldecanoat, Methylisovalerat, Methyloctanoat, Methylpropionat, Nerylacetat, Nerylisobutyrat, n-Octylacetat; Phenethylacetat, Phenethylisobutyrat, Phenethylisovalerat, Phenethylpropionat, Phenyl-propyl-acetat, Phenyl-propylhexanoat, n-Propylacetat, n-Propylformiat und/oder n-Propylisobutyrat.

Zu den bevorzugt einzusetzenden Alkoholen gehören insbesondere Anisylalkohol, Benzylalkohol, 1-Butanol, 1-Hexanol, Isoamylalkohol, Isobutylalkohol, Nerol, Ethanol, Phenethylalkohol, Propanol, 2-Heptanol, 2-Octanol, 3-Octanol, 2-Nonanol und/oder 3-Hexanol.

Zu den natürlichen Extrakten gehören insbesondere Bananenextrakte, Erdbeerextrakte, Kakaoextrakte, Vanilleextrakte, Kaffeeextrakte, Teeextrakte, Nussextrakte, Rumextrakte, Extrakte von Zitrusfrüchten, Kernextrakte, Apfelextrakte und Gewürzextrakte. Diese Extrakte können vielfach kommerziell erhalten werden. Hierzu gehören insbesondere Cocoa Absolute 14620, Cocoa LC 10167, Cocoa P 11197, Cocoa U88; alle erhältlich von Degussa AG. Natürliche Extrakte sind hierbei Extrakte die aus natürlichen Quellen erhalten werden können oder Eigenschaften aufweisen, die diesen Extrakten ähnlich sind.

Gemäß einem weiteren Aspekt der vorliegenden Erfindung können Naturstoffextrakte als niedermolekulare Substanz eingesetzt werden. Naturstoffextrakte sind Extrakte, die aus den Naturstoffen gewonnen werden. Zu den bevorzugten Naturstoffextrakten gehören Zusammensetzungen, die durch Extraktion von Ananas, Apfel, Banane, Bier, Birne, Erdbeere, Zitrusfrüchten, Himbeere, Johannisbeere, Kaffee, Kaffeeöl, Kirsche, Mango, Orangenöl, Passionsfrucht, Rum, Sauerkirsche, Schlehe und/oder Whisky Pure Malt gewonnen werden.

Darüber hinaus können die erfindungsgemäßen Präparate natürliche und synthetische Zusatzstoffe wie Nahrungsmitteladditive umfassen, insbesondere 2-Acetyl-3,5(6)-dimethyl-pyrazin, 2-Acetyl-pyrazin, 2-Acetylthiazol, 2,3-Diethyl-5-methyl-pyrazine, 2,3-Diethyl-pyrazin, 2,3-Dimethyl-pyrazin, 2,5-Dimethyl-pyrazin, 2,6-Dimethyl-pyrazin, 2-Ethyl-3,5-dimethyl-pyrazin, 2-Ethylfuran, 2-Ethyl-3-methyl-pyrazin, 2-Ethyl-5(6)-methyl-pyrazin, 3-Ethylpyridin, 2-Methoxy-3-isobutyl-pyrazin, 2-Methoxy-3(5),(6)-methyl-pyrazin, 2-Methoxy-pyrazin, 2-Methylpyrazin, 2-Pentylfuran, 2,3,5-Trimethyl-pyrazin und/oder Compound 1036 (ein allgemein bekanntes Cognacöl Substitut).

Des Weiteren können die erfindungsgemäßen Präparate Enzym modifizierte diätische Zusatzstoffe (Enzyme Modified Diary Ingredients; EMDI) umfassen. Diese Zusatzstoffe sind für eine Vielzahl von Nahrungsmitteln erhältlich, beispielsweise als Käsegeschmacksstoffe unter der Handelsbezeichnung CPF® Cheese Paste Flavors, NCF® Powderd Cheesed Flavors, BCF® Liquid Blue Cheese Flavors, FDF® Liquid. Cheese Flavors. Des weiteren sind EMDI als Buttergeschmacksstoffe erhältlich, beispielsweise LBO® Butterfat/ Cremepaste Flavors; NBF® Powdered Butterfat Flavors; FDF® /DMF® Liquid Butter Flavors.

Zu den bevorzugten Verbindungen mit einer Peroxidgruppe gehören insbesondere H₂O₂, Persulfate und/oder organische Peroxide.

Zu den Monomeren gehören insbesondere Olefine, beispielsweise 1-Buten, 1-Hexen, Norbornen;
Vinylhalogenide, wie beispielsweise Vinylchlorid, Vinylfluorid, Vinylidenchlorid und Vinylidenfluorid;
Vinylester, wie Vinylacetat;
Styrol, substituierte Styrole mit einem Alkylsubstituenten in der Seitenkette, wie z. B. α-Methylstyrol und α-Ethylstyrol, substituierte Styrole mit einem Alkylsubstitutenten am Ring, wie Vinyltuluol und p-Methylstyrol, halogenierte Styrole, wie beispielsweise Monochlorstyrole, Dichlorstyrole, Tribromstyrole und Tetrabromstyrole;
Heterocyclische Vinylverbindungen, wie 2-Vinylpyridin, 3-Vinylpyridin, 2-Methyl-5-vinylpyridin, 3-Ethyl-4-vinylpyridin, 2,3-'Dimethyl-5-vinylpyridin, Vinylpyrimidin, Vinylpiperidin, 9-Vinylcarbazol, 3-Vinylcarbazol, 4-Vinylcarbazol, 1-Vinylimidazol, 2-Methyl-1-vinylimidazol, N-Vinylpyrrolidon, 2-Vinylpyrrolidon, N-Vinylpyrrolidin, 3-Vinylpyrrolidin, N-Vinylcaprolactam, N-Vinylbutyrolactam, Vinyloxolan, Vinylfuran, Vinylthiophen, Vinylthiolan, Vinylthiazole und hydrierte Vinylthiazole, Vinyloxazole und hydrierte Vinyloxazole;
Vinyl- und Isoprenylether; Maleinsäure und Maleinsäurederivate, wie beispielsweise Mono- und Diester der Maleinsäure, Maleinsäureanhydrid, Methylmaleinsäureanhydrid, Maleinimid, Methylmaleinimid; Fumarsäure und Fumarsäurederivate, wie beispielsweise Mono- und Diester der Fumarsäure;
Diene wie beispielsweise Divinylbenzol;
sowie Acrylate und Methacrylate, die nachfolgend als (Meth)acrylate bezeichnet werden.

Diese umfassen beispielsweise
(Meth)acrylate, die sich von gesättigten Alkoholen ableiten, wie Methyl(meth)acrylat, Ethyl(meth)acrylat, n-Propyl(meth)acrylat, iso-Propyl(meth)acrylat, n-Butyl(meth)acrylat, tert-Butyl(meth)acrylat, Pentyl(meth)acrylat, Hexyl(meth)acrylat, 2-Ethylhexyl(meth)acrylat, Heptyl(meth)acrylat, 2-tert.-Butylheptyl(meth)acrylat, Octyl(meth)acrylat, 3-iso-Propylheptyl(meth)acrylat, Nonyl(meth)acrylat, Decyl(meth)acrylat, Undecyl(meth)acrylat, 5-Methylundecyl(meth)acrylat, Dodecyl(meth)acrylat, 2-Methyldodecyl(meth)acrylat, Tridecyl(meth)acrylat, 5-Methyltridecyl(meth)acrylat, Tetradecyl(meth)acrylat, Pentadecyl(meth)acrylat, Hexadecyl(meth)acrylat, 2-Methylhexadecyl(meth)acrylat, Heptadecyl(meth)acrylat, 5-iso-Propylheptadecyl(meth)acrylat,
4-tert.-Butyloctadecyl(meth)acrylat,
5-Ethyloctadecyl(meth)acrylat,
3-iso-Propyloctadecyl(meth)acrylat, Octadecyl(meth)acrylat, Nonadecyl(meth)acrylat, Eicosyl(meth)acrylat, Cetyleicosyl(meth)acrylat, Stearyleicosyl(meth)acrylat, Docosyl(meth)acrylat und/oder Eicosyltetratriacontyl-(meth)acrylat;
Cycloalkyl(meth)acrylate, wie Cyclopentyl(meth)acrylat, 3-Vinylcyclohexyl(meth)acrylat, Cyclohexyl(meth)acrylat, Bornyl(meth)acrylat;
(Meth)acrylate, die sich von ungesättigten Alkoholen ableiten, wie 2-Propinyl(meth)acrylat, Allyl(meth)acrylat, Vinyl(meth)acrylat und/oder Oleyl(meth)acrylat;
Aryl(meth)acrylate, wie Benzylmethacrylat oder Phenylmethacrylat, wobei die Arylreste jeweils unsubstituiert oder bis zu vierfach substituiert sein können;
Methacrylate von halogenierten Alkoholen, wie 2,3-Dibromopropylmethacrylat,
4-Bromophenylmethacrylat,
1,3-Dichloro-2-propylmethacrylat,
2-Bromoethylmethacrylat,
2-Iodoethylmethacrylat,
Chloromethylmethacrylat.

Des Weiteren können bevorzugte niedermolekulare Substanzen aus dem Bereich der Detergenzien sowie Additive, die in Haarwaschmitteln sowie Haarfärbemitteln eingesetzt werden, beispielsweise Acetaminophen, acetylierter Lanolinalkohol, Achillea Millefolium, Aesculus Hippocastanum, Agave Rigida, Aloe Barbadensis, Alumina, Aluminumchlorhydrat, Aluminumformiat, Aluminumhydroxid, Magnesiumhydroxid, Stearate, insbesondere Aluminiumstearat und Magnesiumstearat, Aluminumsilikat, Aluminumtristearat, Aminomethylpropanol, Ammoniak, Ammoniumbicarbonat, Ammoniumsulfat, Ammoniumthioglykolsäure, Ammoniumthiolactate, Amodimethicone, Anthemis Nobilis, Arachis Hypogaea.

Zu den bevorzugten Katalysatoren gehören insbesondere Metall umfassende Katalysatoren die Hydrierung bzw. Hydrogenierung eingesetzt werden, beispielsweise zur Härtung von Fettsäuren, Nitrilen und Polyolhydrogenierung, Hydrogenierung von Harzen, Aromaten und Weißölen; selektive Hydrogenierung von Acetylenen und Dienen sowie selektive Hydrogenierung von AMS zu Cumol eingesetzt werden.

Des Weiteren gehören hierzu Katalysatoren, die zur Oxidation eingesetzt werden, beispielsweise zur selektiven Oxidation zur Herstellung von Ethylenoxid und Vinylacetatmonomeren; Reinigung von Abgasen aus Prozessen beispielsweise zum Entfernen von CO und VOCs; Reinigung von Abgasen, die halogenierte Kohlenwasserstoffe umfassen; sowie die Katalysatoren zur Reinigung von technischen Gasen.

Diese Katalysatoren umfassen im Allgemeinen Metalle, beispielsweise Nickel, Kobalt, Kupfer, Molybdän, Chrom, Eisen, sowie Platinmetalle, beispielsweise Rhodium, Palladium, Platin. Derartige Katalysatoren sind an sich bekannt und können vielfach kommerziell erhalten werden. Insbesondere gehören hierzu Produkte unter der Handelsbezeichnung catASium® sowie cataXium® von Degussa.

Beispiele für diese Katalysatoren sind unter anderem
(-)-2,3-Bis[(2R, 5R)-2, 5-Dimethylphospholanyl]maleinsäureanhydrid (1,5-cyclooctadien)rhodium(I) tetrafluoroborat (catASium® M(R)Rh);
(-)-2,3-bis[(2R,5%)-2,5-dimethylphospholanyl]maleinsäure-N-methylimid (1,5-cyclooctadien rhodium(I) tetrafluoroborat (catASium® MN(R)Rh);
(+)-2,3-bis[(2S,5S)-2,5-dimethylphospholanyl] maleinsäureanhydrid (1,5-cyclooctadien) rhodium(I) tetrafluoroborat (catASium® M(S)Rh);
(+)-2,3-Bis[(2S,5S)-2,5-dimethylphospholanyl] maleinsäure-N-methylimide (1,5-cyclooctadien) rhodium(I) tetrafluoroborat (catASium® MN(S)Rh);
(+)-(3R,4R)-Bis(diphenylphosphino).-1 -benzylpyrrolidin (catASium® D(R));
(+)-(3R,4R)-Bis(diphenylphosphino)-1 -benzylpyrrolidin (1,5-cyclooctadien) rhodium(I) tetrafluoroborat (catASium® D (R) Rh) ;
   Butyldi-1-adamantylphosphin (cataCXium® A);
   Benzyldi-1-adamantylphosphin (cataCXium® ABn);
   trans-Di(mu-acetato)bis[o-(di-o-tolylphosphino)benzyl]dipalladium (II) (cataCXium® C);
   N-Phenyl-2-(dicyclohexylphosphino)pyrrol (cataCXium® PCy); N-Phenyl-2-(di-t-butylphosphino)pyrrol (cataCXium® PtB); 1-(Methoxyphenyl)-(dicyclohexylphosphino)pyrrol (cataCXium® POMeCy) ;
   1-(2,4,6-Trimethylphenyl)-2-(dicyclohexylphosphino)imidazol (cataCXium® PICy).

In Bezug auf die Katalysatoren ermöglichen die erfindungsgemäßen Präparate eine besonders lange und stabile Haltbarkeit dieser, sowie eine besonders einfache Handhabung. Des Weiteren können die Katalysatoren über einen besonders langen Zeitraum in die Reaktionsmischung freigesetzt werden.

Die zuvor dargelegten niedermolekularen Substanzen können einzeln oder als Mischung von zwei, drei oder mehr eingesetzt werden. Hierbei können die Mischungen niedermolekulare Substanzen der gleichen Klasse oder von unterschiedlichen Klassen umfassen. So kann beispielsweise eine Kombination als niedermolekulare Substanz eine Mischung umfassen, die ein Vitamin und ein Geschmacksstoff aufweist.

Zur Herstellung der erfindungsgemäßen Präparate können die niedermolekularen Verbindungen sowie die hyperverzweigten Polymere miteinander kombiniert werden. Hierzu sind verschiedene Methoden, insbesondere Koazervation, RESS-, GAS- und/oder PGSS-Verfahren sowie Prozesse unter Verwendung von Koaxialdüsen, Sprühtrocknung Wirbelschichtcoating sowie Mikroverkapselung geeignet.

Gemäß bevorzugten Verfahren wird ein komprimiertes Gas verwendet oder es treten mindestens zwei flüssige Phasen auf, wobei beide Phasen die niedermolekulare Substanz enthalten und mindestens eine Flüssigphase ein hyperverzweigtes Polymer enthält.

Beim RESS Verfahren (Rapid Expansion of Supercritical Solution) wird ein Gemisch umfassend ein überkritisches Fluid und eine darin gelöste Substanz plötzlich entspannt. Eine nähere Erläuterung dieses Verfahrens mit weiteren Nachweisen ist in Gamse et al., Chemie Ingenieur Technik 77 (2005), No. 6, Seiten 669 bis 679 dargestellt.

Bei GAS-Verfahren wird zu einer verdünnten Lösung einer niedermolekularen Substanz in Gegenwart von hyperverzweigten Polymeren ein gasförmiges oder überkritisches Fällungsmittel hinzugegeben. Bevorzugt kann dieses Verfahren auch kontinuierlich betrieben werden. Diese Verfahren wurden insbesondere von Gamse et al., Chemie Ingenieur Technik 77 (2005), No. 6, Seiten 669 bis 679 und Tom, J.W.; Lim, G.B.; Debendetti, P.G.; Prod'homme, R.K.Supercritical Fluid Engineering Science, Washington DC 1993: Brennecke, J.F.; Kiran, E.; American Chemical Society: 1993, dargelegt.

Bei den PGSS-Verfahren wird ein komprimiertes Gas zu einer Lösung oder einer Schmelze hinzugefügt. Die gasgesättigte Mischung wird anschließend mit einer Düse expandiert, wobei feste Partikel gebildet werden. Die PGSS-Verfahren wurden unter anderem von

Gamse et al., Chemie Ingenieur Technik 77 (2005), No. 6, Seiten 669 bis 679; Pérez de Diego, Y. Production of Controlled Drug Delivery Microparticles using Supercritical CO2 2004**;** und
Shariati, A.; Peters, C.J. Recent developments in particle design using supercritical fluids. Current Opinion in Solid State & Materials Science 2003, 7 (4-5), 371-383
beschrieben.

Des Weiteren kann das erfindungsgemäße Präparat bevorzugt durch PCA-Verfahren (Precipitation with Compressed Fluid Antisolvent) erhalten werden, wobei eine Lösemittel/Feststoff-Lösung in eine Hochdruckkammer versprüht, und der Phasenkontakt findet in der Sprühkammer zwischen den feinen Tropfen und dem überkritischen Fluid statt, wodurch die feinen Teilchen entstehen (vgl. Gamse et al., Chemie Ingenieur Technik 77 (2005), No. 6, Seiten 669 bis 679).

Darüber hinaus können die Präparate gemäß der vorliegenden Erfindung vorzugsweise mittels SEDS-Verfahren (Solution Enhanced Dispersion by Supercritical Fluids) hergestellt werden. Beim SEDS-Verfahren wird ebenfalls ein Flüssigkeitsstrom erzeugt, der die niedermolekulare Substanz sowie das hyperverzweigte Polymer enthält. Dieser Flüssigkeitsstrom wird bereits in der Düse mit dem überkritischen Fluid in Kontakt gebracht. Durch diese Verfahrensvariante können besonders kleine Teilchen erhalten werden (vgl. Gamse et al., Chemie Ingenieur Technik 77 (2005), No. 6, Seiten 669 bis 679).

Des Weiteren können die erfindungsgemäßen Präparate durch CPF-Verfahren erhalten oder weiterverarbeitet werden. Beim CPF-Verfahren (Concentrates Powder Form) erfolgt keine Veränderung der Partikelgröße. Ziel dieses Verfahrens ist, eine hohe Konzentration einer Flüssigkeit in einem Feststoff zu erreichen. Die Flüssigkeiten können hierbei mit einem geeigneten Gas, beispielsweise CO₂ entweder in einem statischen Mischer vor dem Versprühen in Kontakt gebracht oder bei hochviskosen Substanzen eingelöst werden, wodurch eine drastische Reduktion der Viskosität erzielt werden kann. Die mittels einer Düse feinsten Flüssigkeitstropfen prallen auf den parallel zudosierten Feststoff und haften an diesem an, so dass Agglomerate entstehen können, die hohe Flüssigkeitsanteile aufweisen. Des Weiteren kann der Feststoff mit dieser Flüssigkeit durchtränkt werden (vgl. Gamse et al., Chemie Ingenieur Technik 77 (2005), No. 6, Seiten 669 bis 679).

Bei den zuvor beschriebenen Verfahren, die mit Hochdruck arbeiten, insbesondere den RESS-, GAS- und/oder PGSS-Verfahren kann die Verkapselung mit Vorteil bei Temperaturen in einem Bereich von -20°C bis 250°C, vorzugsweise 0°C bis 180°C und besonders bevorzugt 10°C bis 150°C erfolgen. Der Druck bei diesen Verfahren kann über einen weiten Bereich variiert werden, wobei diese Hochdruckverfahren vorzugsweise in einem Bereich von 0,5 bar bis 400 bar, besonders bevorzugt 1 bar bis 200 bar und ganz besonders bevorzugt 1 bar bis 100 bar durchgeführt werden können.

Bei den Hochdruckverfahren kann die Konzentration der niedermolekularen Komponente im hyperverzweigten Trägerpolymer vorzugsweise im Bereich von 0,5 Massen% bis 95 Massen%, besonders bevorzugt 1 Massen% bis 80 Massen% und ganz besonders bevorzugt 5 Massen% bis 50 Massen% liegen.

Bei der Koazervation werden die Partikel durch Fällung aus einer Lösung von hyperverzweigtem Polymer und niedermolekularer Substanz gebildet.

Man unterscheidet im Allgemeinen zwischen einfacher und komplexer Koazervation, sowie zwischen wässriger und organischer Phasentrennung (R. Arshady, "Microspheres and microcapsules - A survey of manufacturing techniques, Part III: Coacervation", Polymer Engineering and Science 30 (1990) 905ff). Bei der einfachen Koazervation kommt eine kolloidale Komponente, z. B. Gelatine, und bei der komplexen Koazervation zwei gegensätzlich geladene kolloidale Komponenten, z.B. Gelatine und Gum Arabicum, zum Einsatz. Das Prinzip der Koazervation besteht darin, dass z.B. eine wässrige Gelatinelösung durch Zugabe von Ethanol in ein Zweiphasen-System überführt wird, das aus einer Gelatine-reichen (Koazervat) und eine Gelatine-armen Phase besteht. Dies ist einer Polymerfraktionierung sehr ähnlich, allerdings entstehen in diesem Fall unter Einwirkung von Scherkräften Mikropartikel mit einer durchschnittlichen Größe von 2 - 5000 Mikrometer.

Die Herstellung von Mikrokapseln durch Koazervation lässt sich vielfach in drei Schritte unterteilen:
(1) Erzeugung (dreier) nicht mischbarer Phasen,
(2) Abscheidung des Kolloids als Kapselhülle und
(3) Verfestigung der Kapselhülle.

Die drei nicht mischbaren Phasen umfassen ein äußeres Medium, ein Kernmaterial und ein Kapselhüllmaterial. Das Kapselhüllmaterial ist im äußeren Medium gelöst und das Kernmaterial ist darin dispergiert. Durch Einwirkung eines äußeren Stimulus (Temperatur, pH, Elektrolyte) wird das Kapselhüllmaterial im äußeren Medium unlöslich und legt sich an der Grenzfläche zum dispergierten Kernmaterial ab. Nach Filtration wird die Kapselhülle schließlich durch Einwirkung von Wärme, Vernetzung oder Lösemittelentzug ausgehärtet oder durch Sprühtrocknung oder Gefriertrocknung getrocknet.

Vorzugsweise wird die Koazervation bei Temperaturen im Bereich von -20°C bis 150°C, besonders bevorzugt 0°C bis 00°C und ganz besonders bevorzugt 10°C bis 90°C durchgeführt.

Der Druck bei dem die Koazervation stattfindet unterliegt keiner besonderen Beschränkung, Vielfach kann die Koazervation bei einem Druck im Bereich von 10 mbar bis 10 bar, vorzugsweise 200 mbar bis 5 bar und besonders bevorzugt 500 mbar bis 3 bar durchgeführt werden.

Vorzugsweise kann die Konzentration der niedermolekularen Komponente im hyperverzweigten Trägerpolymer im Bereich von 0,5 Massen% bis 95 Massen%, besonders bevorzugt 1 Massen% bis 80 Massen% und ganz besonders bevorzugt 5 Massen% bis 50 Massen% liegen, ohne dass hierdurch eine Beschränkung erfolgen soll.

Dieses Verfahren wurde unter anderem von
R. Arshady, "Microspheres and microcapsules - A survey of manufacturing techniques, Part III: Coacervation", Polymer Engineering and Science 30 (1990) 905ff. ;
Jain, R.A. The manufacturing techniques of various drug loaded biodegradable poly(lactide-co-glycolide) (PLGA) devices. Biomaterials 2000, 21 (23), 2475-2490;
Jung, J.; Perrut, M. Particle design using supercritical fluids: Literature and patent survey. Journal of Supercritical Fluids 2001, 20 (3), 179-219; und
Subramaniam, B.; Rajewski, R.A.; Snavely, K. Pharmaceutical processing with supercritical carbon dioxide. Journal of Pharmaceutical Sciences 1997, 86 (8), 885- 890
   beschrieben.

Die Sprühtrocknung ist ein kontinuierlich durchführbares Verfahren zur Trocknung von Lösungen, Suspensionen oder pastösen Massen. Dieses Verfahren ist weithin bekannt, wobei Anlagen zur Durchführung des Verfahrens kommerziell erhältlich sind. Im Allgemeinen wird Mittels einer Düse (durch Flüssigkeitsdruck oder Pressluft bzw. Inertgas betrieben) oder rotierenden Sprühscheiben (4000-50000 U/min) das zu trocknende Gut in einen Heißluftstrom (Temperaturen je nach Apparatur bis zu 220°) eingebracht der es in Bruchteilen einer Sekunde zu einem feinen Pulver trocknet. Die Heißluft kann in Richtung mit dem Sprühstrahl oder gegen den Sprühstrahl strömen (Gleichstrom-, Gegenstromverfahren), je nach Bauart oder Verwendungszweck. Die Sprüheinrichtung befindet sich am oberen Teil eines Sprühturms, das anfallende Trockengut wird meist durch einen Zyklonabscheider vom Luftstrom getrennt und kann dort entnommen werden.

Unter Mikroverkapselung versteht man das Einbetten mindestens einer Substanz (Wirkstoff) mit Hilfe mindestens einer zweiten (Hüllmaterial). Erstere kann oft aus verschiedenen Gründen (Löslichkeit, Reaktivität, Stabilität etc.) nicht direkt eingesetzt werden, oder es sollen bestimmte Wirkungen durch die Mikroverkapselung erzielt werden (z.B. Freisetzungskurven für Controlled Release, Alleinstellungsmerkmale etc.).

In der Praxis gibt es eine Reihe von Ansätzen zur Mikroverkapselung, die im Wesentlichen auf zwei Methoden hinauslaufen nämlich die Matrixverkapselung und die Kern-Hülle-Verkapselung.

Für beide Methoden sind kommerzielle Lösungen erhältlich. Dazu werden im Allgemeinen individuell auf die Wirkstoffe zugeschnittene Rezepturen und Verfahren verwendet. Ein kommerzieller Anbieter dieser Lösungen ist BRACE GmbH, Alzenau.

Gemäß einem bevorzugten Aspekt der vorliegenden Erfindung können die erfindungsgemäßen Präparate durch ein Verfahren erhalten werden, umfassend die Schritte

Herstellen einer Polymerschmelze umfassend mindestens ein hyperverzweigtes Polymer und mindestens eine niedermolekulare Substanz,

Einleiten der Polymerschmelze in eine zweite flüssige Phase, in der das hyperverzweigtes Polymer schwer löslich ist, wobei die zweite flüssige Phase eine Verfestigungstemperatur aufweist, die unterhalb der Verfestigungstemperatur des hyperverzweigtes Polymers liegt,

Dispergieren der Polymerschmelze in der zweiten flüssigen Phase bei einer Temperatur, die größer oder gleich der Verfestigungstemperatur des hyperverzweigtes Polymer ist und Verfestigen der in der zweiten flüssigen Phase dispergierten Polymerschmelze.

Gemäß dem bevorzugten Verfahren wird eine Polymerschmelze hergestellt. Der Begriff Polymerschmelze bezeichnet einen fließfähigen Zustand einer Zusammensetzung, die mindestens ein hyperverzweigtes Polymer und mindestens eine niedermolekulare Substanz umfässt. Vorzugsweise liegt die Viskosität der Polymerschmelze im Bereich von 50 mPa*s bis 5000 Pa*s , besonders bevorzugt im Bereich von 100 mPa*s bis 1000 Pa*s, wobei diese Größe mittels Rotationsviskosimetrie bestimmt werden kann. Der fließfähige Zustand ist hierbei unter anderem von der Temperatur abhängig. Bevorzugt werden die zuvor genannten Viskositätsbereiche der Polymerschmelze bei einer Temperatur im Bereich von 10 bis 200 °C, besonders bevorzugt im Bereich von 50 bis 150 °C gemessen. Gemäß einem besonderen Aspekt der vorliegenden Erfindung kann die Viskosität der Polymerschmelze im Bereich von 100 mPa*s bis 1000 Pa*s liegen, wobei diese Größe mittels Rotationsviskosimetrie bei 110°C und 30 s⁻¹ zwischen zwei 20 mm Platten gemessen werden kann.

Die niedermolekulare Substanz wird vorzugsweise fein in der Polymerschmelze verteilt. Hierzu können bekannte Vorrichtungen, wie zum Beispiel Rührwerke, die einen Rührkessel mit Propeller-, Scheiben-, Zahnscheiben-, Anker- , Wendel-, Blatt-, Schaufel-, Schrägblatt-, Kreuzblatt-, Schrauben-, MIG-, INTERMIG-, ULTRA-TURRAX, Schnecken-, Band-, Finger-, Korb-, Impeller-Rührer umfassen, sowie Dispergatoren und Homogenisatoren, die unter anderem mit Ultraschall arbeiten können, eingesetzt werden. Die Vorrichtungen können im Allgemeinen mindestens eine Welle aufweisen, an der wiederum vorzugsweise 1 bis 5 Rührelemente angebracht sein können.

Hierbei kann beispielsweise eine Lösung, eine Suspension oder eine Dispersion entstehen, wobei die Teilchengröße der verteilt vorliegenden Phase vorzugsweise höchstens 5000 µm, besonders bevorzugt höchstens 1000 µm beträgt, falls die niedermolekulare Substanz partikelförmig vorliegt.

Die hierzu notwendigen Parameter sind von den zuvor dargelegten Vorrichtungen abhängig. Vorzugsweise kann die Rührgeschwindigkeit im Bereich von 10 bis 25000 Umdrehungen pro Minute, besonders bevorzugt im Bereich von 20 bis 10000 Umdrehungen pro Minute liegen.

Die Temperatur bei der die Polymerschmelze hergestellt wird, kann ebenfalls in einem weiten Bereich liegen, wobei diese unter anderem von der Verfestigungstemperatur des hyperverzweigten Polymers abhängig ist. Vorzugsweise liegt die Temperatur im Bereich von -20°C bis 250°C, besonders bevorzugt im Bereich von 0°C bis 200°C. Gemäß einem besonderen Aspekt der vorliegenden Erfindung wird die Temperatur beim Herstellen der Polymerschmelze 10°C bis 200°C, besonders bevorzugt im Bereich 15°C bis 150°C oberhalb der Verfestigungstemperatur des hyperverzweigten Polymers gewählt. Der beim Herstellen der Polymerschmelze eingesetzte Druck ist ebenfalls unkritisch, wobei dieser vielfach von der Art der niedermolekularen Substanz und der Verfestigungstemperatur des hyperverzweigten Polymers abhängig ist. Beispielsweise kann der Druck im Bereich von 0,1 mbar bis 200bar, bevorzugt im Bereich von 10 mbar bis 100 bar gewählt werden.

Gemäß einem besonderen Aspekt der vorliegenden Erfindung wird der Polymerschmelze vorzugsweise kein Lösungsmittel, insbesondere kein organisches Lösungsmittel zugegeben, wobei besonders bevorzugte Polymerschmelzen kein Lösungsmittel umfassen. Als Lösungsmittel wird hierbei eine Substanz verstanden, in der das hyperverzweigte Polymer löslich ist und die während des Herstellungsprozesses abgetrennt werden muss, da diese Verbindung nicht in den Präparaten enthalten sein sollte. In diesem Zusammenhang ist festzuhalten, dass viele der zuvor dargelegten niedermolekularen Substanzen Eigenschaften eines Lösungsmittels haben können. Diese Substanzen sind jedoch ein gewollter Bestandteil der Präparate, so dass diese Verbindungen in Rahmen der vorliegenden Erfindung kein Lösungsmittel darstellen. Dementsprechend ist die Verwendung von Lösungsmittel zur Durchführung des Verfahrens nicht notwendig. Andererseits werden einige niedermolekularen Substanzen in gelöster Form geliefert, wobei die hierfür verwendeten Lösungsmittel im Allgemeinen für Verwendung der niedermolekularen Substanz nicht kritisch sind, so dass diese beispielsweise gesundheitlich unbedenklich sind, falls die niedermolekulare Substanz biologisch wirksam ist. Derartige Hilfsstoffe müssen nicht notwendig vor der Herstellung der Polymerschmelze abtrennt werden. Vielmehr können diese Hilfssubstanzen in die Polymerschmelze eingearbeitet werden.

Die zuvor beschriebene Polymerschmelze wird erfindungsgemäß in eine zweite flüssige Phase überführt, in der das hyperverzweigte Polymer schwer löslich ist und die eine Verfestigungstemperatur unterhalb der Verfestigungstemperatur des, hyperverzweigten Polymers aufweist.

Der Begriff "schwer löslich" bedeutet, dass die Löslichkeit des hyperverzweigten Polymers in der zweiten flüssigen Phase möglichst gering sein sollte. Die Löslichkeit ist vielfach von der Temperatur abhängig. Dementsprechend können die Dispergierbedingungen vielfach so gewählt werden, dass ein möglichst geringer Anteil des hyperverzweigten Polymers in der zweiten flüssigen Phase gelöst wird. Vorzugsweise weist das hyperverzweigten Polymer eine Löslichkeit nach der Kolbenmethode bei der Dispergiertemperatur von höchstens 20 Massenprozent, bevorzugt höchstens 10 Massenprozent in der zweiten flüssigen Phase auf.

Die zweite flüssige Phase weist eine Verfestigungstemperatur unterhalb der Verfestigungstemperatur des hyperverzweigten Polymers auf. Im Allgemeinen ergibt sich diese Temperatur aus der Schmelztemperatur oder der Glasübergangstemperatur des Hauptbestandteils der zweiten flüssigen Phase, wobei Gefrierpunktserniedrigungen durch Hilfs- oder Zusatzstoffe bzw. durch die Verwendung von Stoffgemischen auftreten können. Diese Größe kann aus DSC-Messungen erhalten werden, wobei die Schmelzpunkte bzw. Gefrierpunkte der üblichen Hauptbestandteile der zweiten flüssigen Phase in Nachschlagewerken aufgeführt sind.

Vorzugsweise umfasst die zweite flüssige Phase gesundheitlich unbedenkliche Substanzen, die besonders bevorzugt eine Zulassung gemäß Community Register of Feed Additives pursuant to Regulation (EC) No 1831/2003, Rev. 4 Status: Released 29 May 2006 (FDA) aufweisen.

Bei Verwendung von hydrophoben hyperverzweigten Polymeren wird in der zweiten flüssigen Phase vorzugsweise mindestens eine hydrophile Substanz als Hauptbestandteil eingesetzt. Zu den hydrophilen Substanzen, die als Hauptbestandteil in der zweiten flüssigen Phase enthalten sein können, gehören insbesondere Wasser und Alkohole mit 1 bis 7, vorzugsweise 1 bis 4 Kohlenstoffatomen, insbesondere Methanol, Ethanol, Propanol und/oder Butanol.

Die zweite flüssige Phase kann neben dem Hauptbestandteil zusätzliche Hilfsstoffe, insbesondere Dispergatoren und Stabilisatoren umfassen. Diese Hilfsstoffe sind in der Fachwelt bekannt, wobei Dispergatoren einer Aggregation der Partikel entgegenwirken. Hierzu gehören insbesondere Emulgatoren, Schutzkolloide und Tenside, die jeweils entsprechend den eingesetzten hyperverzweigten Polymeren, niedermolekularer Substanz und Hauptbestandteil der zweiten flüssigen Phase eingesetzt werden können. Zu den bevorzugten Tensiden gehören insbesondere anionische Tenside, wie Laurylethersulfat, kationische Tenside und nichtionische Tenside, wie beispielsweise Polyvinylalkohole und ethoxylierte Fettalkohole. Stabilisatoren können für eine Vielzahl von Anwendungen eingesetzt werden, wobei diese Hilfsstoffe einen erwünschten, instabilen Zustand erhalten bzw. stabilisieren. Hierzu gehören insbesondere Absetzverhinderungsmittel, wie Pektine und/oder Karragenan.

Bevorzugt umfasst die zweite flüssige Phase 60 bis 100 Gew.-% Hauptbestandteil, beispielsweise die zuvor dargelegten hydrophilen Substanzen, wie Wasser oder Alkohole mit bis zu 4 Kohlenstoffatomen. Weiterhin kann die zweite flüssige Phase 0 bis 40 Gew.-% Hilfssubstanzen enthalten, insbesondere 0 bis 20 Gew.-% Emulgatoren und 0 bis 20 Gew.-% Stabilisatoren.

Die in die zweite flüssige Phase eingebrachte Polymerschmelze wird bei einer Temperatur, die größer oder gleich der Verfestigungstemperatur des hyperverzweigten Polymers ist, dispergiert.

Die Verfestigungstemperatur des hyperverzweigten Polymers bezeichnet im Rahmen der vorliegenden Erfindung die Temperatur bei der das hyperverzweigte Polymer fest wird, so dass Polymerpartikel bei dieser Temperatur nicht mehr ohne äußere Einwirkungen zu größeren Aggregaten agglomerieren. Je nach Aufbau und Kristallisationseigenschaften kann die Verfestigungstemperatur beispielsweise durch die Glasübergangstemperatur bzw. die Schmelztemperatur des hyperverzweigten Polymers gegeben sein, die beispielsweise durch DSC-Verfahren (Differential Scanning Calorimetry; Dynamische Differenzkalorimetrie) bestimmt werden können. Hierbei ist festzuhalten, dass teilkristalline Polymere sowohl eine Glasübergangstemperatur als auch eine Schmelztemperatur zeigen können, wobei in diesem Fall die Temperatur ausschlaggebend ist, bei der die Partikel keine Agglomeration zeigen. Falls die Oberfläche im Wesentlichen kristallin ist, so ist vielfach der Schmelzpunkt dieser Bestandteile entscheidend.

Dispergieren bedeutet in diesem Zusammenhang, dass die mindestens eine niedermolekulare Substanz umfassende Polymerschmelze fein in der kontinuierlichen zweiten flüssigen Phase verteilt wird. Das Dispergieren kann hierbei mit bekannten Geräten und Vorrichtungen, wie zum Beispiel Rührwerken, die einen Rührkessel mit Propeller-, Scheiben-, Zahnscheiben-, Anker-, Wendel-, Blatt-, Schaufel-, Schrägblatt-, Kreuzblatt-, Schrauben-, MIG-, INTERMIG-, ULTRA-TURRAX, Schnecken-, Band-, Finger-, Korb-, Impeller-Rührer umfassen sowie Dispergatoren und Homogenisatoren, die unter anderem mit Ultraschall arbeiten können, durchgeführt werden. Die Vorrichtungen können im Allgemeinen mindestens eine Welle aufweisen, an der wiederum vorzugsweise 1 bis 5 Rührelemente angebracht sein können.

Die Dauer und der Energieeintrag des Dispergierens sind hierbei von der gewünschten Partikelgröße und Partikelgrößenverteilung abhängig. Dementsprechend kann die Dauer des Dispergierens in einem weiten Bereich gewählt werden. Vorzugsweise wird das Dispergieren für eine Zeitdauer im Bereich von 1 Sekunde bis 5 Stunden, besonders bevorzugt im Bereich von 10 Sekunden bis 2 Stunden durchgeführt.

Gemäß einem besonderen Aspekt kann die Polymerschmelze vorzugsweise 10 bis 99,99 Gew.-%, besonders bevorzugt 50 bis 99,00 Gew.-% und ganz besonders bevorzugt 70 bis 90 Gew.-% hyperverzweigtes Polymer umfassen. Des Weiteren kann die Polymerschmelze vorzugsweise 0,01 Gew.-% bis 90 Gew.-%, vorzugsweise 1 Gew.-% bis 50 Gew. -% und ganz besonders bevorzugt 10 Gew.-% bis 30 Gew.-% niedermolekulare Substanz umfassen.

Gemäß einem besonderen Aspekt der vorliegenden Erfindung kann die Newtonzahl beim Dispergieren vorzugsweise im Bereich von 0,1 bis 1000, besonders bevorzugt im Bereich von 0,4 bis 800 liegen.

Entsprechend einer besonderen Ausführungsform des erfindungsgemäßen Verfahrens kann die Reynoldszahl beim Dispergieren vorzugsweise im Bereich von 1 bis 10⁷, besonders bevorzugt im Bereich von 10 bis 10⁶ liegen.

Die hierzu notwendigen Parameter sind von den zuvor dargelegten Vorrichtungen abhängig. Vorzugsweise kann die Rührgeschwindigkeit im Bereich von 10 bis 25000 Umdrehungen pro Minute, besonders bevorzugt im Bereich von 20 bis 10000 Umdrehungen pro Minute liegen.

Hierbei ist die eingesetzte Newtonzahl bzw. die Rührgeschwindigkeit von der gewünschten Partikelgröße und Partikelgrößenverteilung abhängig. Je mehr Energie zugeführt wird und je länger dispergiert wird, desto kleinere Partikelgrößen können erzielt werden. Eine enge Partikelgrößenverteilung kann ebenfalls durch eine hohe Dispergierenergie und eine lange Dispergierzeit erzielt werden. Andererseits sind lange Dispergierzeiten und hohe Dispergierenergien vielfach mit zusätzlichen Kosten verbunden.

Die Temperatur bei der die Polymerschmelze in der zweiten flüssigen Phase dispergiert wird, kann ebenfalls in einem weiten Bereich liegen, wobei diese unter anderem von der Verfestigungstemperatur des hyperverzweigten Polymers abhängig ist. Vorzugsweise liegt die Temperatur im Bereich von -20°C bis 250°C, besonders bevorzugt im Bereich von 0 bis 200°C. Der beim Dispergieren der Polymerschmelze eingesetzte Druck ist ebenfalls unkritisch, wobei dieser vielfach von der Art der niedermolekularen Substanz und der Verfestigungstemperatur des hyperverzweigten Polymers abhängig ist. Beispielsweise kann der Druck im Bereich von 10 mbar bis 200 bar, bevorzugt im Bereich von 100 mbar bis 100 bar gewählt werden.

Die Temperatur beim Dispergieren ist größer oder gleich der Verfestigungstemperatur des hyperverzweigten Polymers. Vorzugsweise liegt die Dispergiertemperatur 1 °C bis 200 °C, besonders bevorzugt 5 °C bis 150°C und ganz besonders bevorzugt 10 bis 50 °C oberhalb der Verfestigungstemperatur des hyperverzweigten Polymers.

Das Gewichtsverhältnis von Polymerschmelze zur zweiten flüssigen Phase kann in einem weiten Bereich liegen. Vorzugsweise liegt dieses Verhältnis im Bereich von 1:1 bis 1:1000, besonders bevorzugt 1:1,5 bis 1:500.

Beim Dispergieren kann die Zusammensetzung beispielsweise 50 bis 99 Gew.-%, bevorzugt 70 bis 98 Gew.-% zweite flüssige Phase und 1 bis 50 Gew.-%, bevorzugt 2 bis 30 Gew.-% Polymerschmelze umfassen.

Nach dem die Polymerschmelze in der zweiten flüssigen Phase dispergiert vorliegt, wird die dispergierte Polymerschmelze verfestigt. Die Verfestigung kann mit bekannten Methoden, beispielsweise durch Zugabe von Salzen bei einer Temperatur leicht oberhalb der Verfestigungstemperatur oder durch Abkühlen erfolgen. Vorzugsweise erfolgt das Verfestigen der Polymerschmelze durch Abkühlen der zweiten flüssigen Phase auf eine Temperatur unterhalb der Verfestigungstemperatur. des hyperverzweigten Polymers.

Die Art der Abkühlung ist unter anderem von der gewünschten Partikelgröße und Partikelgrößenverteilung abhängig. Eine schnelle Abkühlung kann unter anderem zu einer besonders einheitlichen Partikelgrößenverteilung und kleinen Partikeln führen, da eine Aggregation vermieden werden kann. Hierbei ist die Bildung von Aggregaten bei einem großen Abkühlvolumen geringer.

Des Weiteren können die Partikelgrößenverteilung und die Größe der Partikel über Hilfsmittel, wie beispielsweise Dispergatoren und Emulgatoren, beeinflusst werden. Diese Additive können beispielsweise in die zweite Phase hinzu gegeben werden, wobei eine Additivierung der Oberfläche der gebildeten Partikel erzielt werden kann. Diese Additivierung kann auch eine Aggregation der Mikropartikel während des Trocknens oder beim Lagern vermindern.

Je nach Anwendung kann die so erhaltene Zusammensetzung unmittelbar weiter verarbeitet werden, ohne dass eine Aufreinigung, Aufkonzentration oder Abtrennung vorgenommen wird. Gemäß einer besonderen Ausführungsform kann das vorliegende Verfahren den Schritt des Abtrennens der in der zweiten flüssigen Phase gebildeten Mikropartikel aufweisen. Das Abtrennen kann durch bekannte Verfahren, insbesondere durch Filtrieren, Zentrifugieren, Sedimentation, Magnettrennung, Flotation, Sieben oder Dekantieren erfolgen, wobei die Verfahren einzeln oder in Kombination eingesetzt werden können. Hierbei können die Verbindungen der zweiten flüssigen Phase im wesentlichen vollständig abgetrennt werden, so dass getrocknete Mikropartikel erhalten werden oder die Partikel können aufkonzentriert werden, wobei noch größere Mengen an Verbindungen der zweiten flüssigen Phase im Endprodukt enthalten sind. Vorzugsweise kann das Endprodukt mindestens 80 Gew.-% Mikropartikel, besonders bevorzugt mindesten 95 Gew.-% Mikropartikel aufweisen.

Die zum Abtrennen oder Aufkonzentrieren der Mikropartikel verwendbaren Vorrichtungen, nachfolgend auch Separatoren genannt, sind allgemein bekannt. So können unter anderem Zentrifugen, Dekanter, Fliehkraftabscheider, Filter, beispielsweise Schwerkraftfilter, Saugfilter (Vakuumfilter), Druckfilter, Saug-Druck-Filter, Pressfilter, Vakuum-Trommelfilter, Bandfilter, Scheibenfilter, Planfilter, Kammerfilterpresse, Rahmenfilterpresse, Kerzenfilter, Blattfilter, Membranfilterplatte und/oder Siebbandpressen eingesetzt werden.

Die Temperatur beim Abtrennen oder Aufkonzeritrieren kann ebenfalls in einem weiten Bereich liegen, wobei diese unter anderem von der Verfestigungstemperatur des hyperverzweigten Polymers abhängig ist. Um eine Aggregation der Partikel zu vermeiden, sollte die gewählte Temperatur unterhalb der Verfestigungstemperatur des hyperverzweigten Polymers liegen. Vorzugsweise liegt die Temperatur im Bereich von -20°C bis 250°C, besonders bevorzugt im Bereich von -10 °C bis 100 °C. Der beim Abtrennen oder Aufkonzentrieren eingesetzte Druck ist ebenfalls unkritisch, wobei dieser vielfach von der Art der niedermolekularen Substanz und der Verfestigungstemperatur des hyperverzweigten Polymers abhängig ist. Beispielsweise kann der Druck im Bereich von 10 mbar bis 200 bar, bevorzugt im Bereich von 100 mbar bis 100 bar gewählt werden.

Nach dem Abtrennen können die erhaltenen Partikel gewaschen werden. Hierzu können die Partikel mit einer Waschflüssigkeit behandelt werden, um Additivreste und/oder niedermolekulare Substanz, die sich auf der Oberfläche der Partikel befindet von den Partikeln zu trennen. Dementsprechend sollten die Partikel, insbesondere die hyperverzweigten Polymere nicht in der Waschflüssigkeit löslich sein. Andererseits sollte die abzutrennende Substanz, beispielsweise die niedermolekulare Substanz eine möglichst hohe Löslichkeit aufweisen. Zu den bevorzugten hydrophilen Waschflüssigkeiten zählen insbesondere Wasser und/oder Alkohole mit 1 bis 7, vorzugsweise 1 bis 4 Kohlenstoffatomen, insbesondere Methanol, Ethanol, Propanol und/oder Butanol. Diese Flüssigkeiten können einzeln oder auch als Mischung von zwei, drei oder mehr Flüssigkeiten eingesetzt werden.

Die Temperatur beim Waschen kann ebenfalls in einem weiten Bereich liegen, wobei diese unter anderem von der Verfestigungstemperatur des hyperverzweigten Polymers abhängig ist. Um eine Aggregation der Partikel zu vermeiden, sollte die gewählte Temperatur unterhalb der Verfestigungstemperatur des hyperverzweigten Polymers liegen. Vorzugsweise liegt die Temperatur im Bereich von - 20°C bis 250°C, besonders bevorzugt im Bereich von -10 °C bis 100 °C. Der beim Waschen eingesetzte Druck ist ebenfalls unkritisch, wobei dieser vielfach von der Art der niedermolekularen Substanz und der Verfestigungstemperatur des hyperverzweigten Polymers abhängig ist. Beispielsweise kann der Druck beim Waschen im Bereich von 10 mbar bis 200 bar, bevorzugt im Bereich von 100 mbar bis 100 bar gewählt werden.

Die zum Waschen der Partikel verwendbaren Vorrichtungen sind allgemein bekannt. So können hierfür beispielsweise Vorrichtungen eingesetzt werden, die ein Mischgefäß und einen Separator umfassen. Die Mischgefäße umfassen vorzugsweise die zuvor dargelegten Geräten und Vorrichtungen zum Dispergieren. Des Weiteren können die Mischgefäße temperierbar ausgestattet sein. Dementsprechend können diese Mischgefäße Heizelemente oder Kühlelemente umfassen.

In einem weiteren Schritt können die erhaltenen Mikropartikel getrocknet werden. Die zum Trocknen der Mikropartikel verwendbaren Vorrichtungen sind allgemein bekannt. So können unter anderem Trommeltrockner, Taumeltrockner, Tellertrockner, Schneckentrockner, Schaufeltrockner, Zylindertrockner, Walzentrockner, Gefriertrockner, Wirbelschichttrockner, Sprühtrockner, Stromtrockner, Mahltrockner, Hordentrockner, Tunneltrockner, Vakuumtrockner- und/oder Vakuumkontakttrockner eingesetzt werden.

Die Temperatur beim Trocknen kann ebenfalls in einem weiten Bereich liegen, wobei diese unter anderem von der Verfestigungstemperatur des hyperverzweigten Polymers abhängig ist. Um eine Aggregation der Partikel zu vermeiden, sollte die gewählte Temperatur unterhalb der Verfestigungstemperatur des hyperverzweigten Polymers liegen. Vorzugsweise liegt die Temperatur beim Trocknen im Bereich von -20°C bis 150°C, besonders bevorzugt im Bereich von -10 °C bis 100°C. Der beim Trocknen eingesetzte Druck ist ebenfalls unkritisch, wobei dieser vielfach von der Art der niedermolekularen Substanz und der Verfestigungstemperatur des hyperverzweigten Polymers abhängig ist. Beispielsweise kann der Druck im Bereich von 0,1 mbar bis 10 bar, bevorzugt im Bereich von 0,2 mbar bis 2 bar gewählt werden.

Das zuvor beschriebene Verfahren können mit einfachen Anlagen durchgeführt werden, die aus an sich bekannten Komponenten aufgebaut sein können. Geeignete Anlagen umfassen vorzugsweise mindestens zwei Mischgefäße und einen Separator, wobei die Mischgefäße über mindestens eine Zuführung miteinander verbunden sind und das zweite Mischgefäß mit dem Separator verbunden ist. Die im Separator abgetrennte zweite Phase kann vorzugsweise über eine Rückführung in ein Mischgefäß zurückgeführt werden.

Gemäß einer bevorzugten Ausführungsform kann in der Leitung zwischen dem ersten Mischgefäß, in dem die Polymerschmelze hergestellt wird und dem zweiten Mischgefäß, in dem die Polymerschmelze in der zweiten flüssigen Phase dispergiert wird, eine Pumpe vorgesehen sein, die für hochviskose Flüssigkeiten geeignet ist. Zu den bevorzugten Pumpen gehören insbesondere Schraubenpumpen, beispielsweise Schraubenpumpen mit einer, zwei oder drei Schrauben; Schraubenverdichter, Flügelpumpen, Drehkolbenpumpen, Rotationspumpen, Kolbenpumpen, Rotationskolbenpumpen und/oder Schlauchpumpen.

Gemäß einem besonderen Aspekt der vorliegenden Erfindung weist die Anlage vorzugsweise mindestens drei Mischgefäße auf, wobei mindestens zwei Mischgefäße über Zuführungen mit mindestens einem Mischgefäß verbunden sind. Hierbei dient mindestens ein Mischgefäß zum Herstellen der Polymerschmelze, mindestens ein Mischgefäß zum Herstellen der zweiten flüssigen Phase und mindestens ein Mischgefäß zum Dispergieren der Polymerschmelze in der zweiten flüssigen Phase. Die Polymerschmelze und die zweite flüssige Phase können in weiteren separaten Mischgefäßen chargenweise oder kontinuierlich hergestellt werden, um eine kontinuierliche Produktion von Mikropartikeln sicherzustellen.

Vorzugsweise kann die Anlage mindestens einen Trockner aufweisen, der mit dem Separator verbunden ist. Des Weiteren kann die Anlage bevorzugt eine Vorrichtung zum Waschen von Partikeln umfassen.

Das Verfestigen der Polymerschmelze in der Dispersion kann in der Anlage über verschiedene Maßnahmen erzielt werden. Beispielsweise kann in das Mischgefäß, in der die Dispersion hergestellt wurde, abgekühlt werden. Dies kann beispielsweise durch eine externe Kühlung oder durch Zuleiten von Flüssigkeiten erfolgen, die vorzugsweise die gleiche oder eine ähnliche Zusammensetzung aufweist wie die zweite flüssige Phase. Bevorzugt kann hierfür auch ein Wärmeüberträger bzw. Wärmetauscher, ein Mischventil oder ein zusätzliches Mischgefäß eingesetzt werden.

Die Anlage kann Pumpen umfassen, die beispielsweise für den Transport von Flüssigkeiten oder zum Erzeugen von Über- bzw. Unterdruck dienen können. Geeignete Pumpen sind vom jeweiligen Zweck abhängig. Zu den bevorzugten Pumpen gehören beispielsweise Verdrängerpumpen, wie zum Beispiel Schöpfwerke, Förderschnecken, Balgpumpen, Kolbenpumpen, Rotationskolbenpumpen, Zahnradpumpen außen/innenverzahnt, Membranpumpen, Drehschieberpumpen, Zentrifugalpumpe, Schlauchpumpen, Zahnriemenpumpen, Exzenterschneckenpumpen, Schraubenpumpen und Schraubenverdichter und/oder Hydraulischer Widder;
Strömungspumpen, wie zum Beispiel Kreiselpumpe, Axialpumpe, Diagonalpumpe und/oder Radialpumpe;
Blasenpumpe, Wasserstrahlpumpe, Dampfstrahlpumpe, Stoßheber (Hydraulischer Widder), Pferdekopfpumpe (Tiefpumpe);
Vakuumpumpen, wie zum Beispiel Verdrängerpumpe, Treibmittelpumpe, Molekularpumpe, Turbomolekularpumpe, Kryopumpe, Sorptionspumpe, Öldiffusionspumpe.

Derartige Anlagen werden in den nachfolgend näher beschriebenen Figuren beispielhaft erläutert.

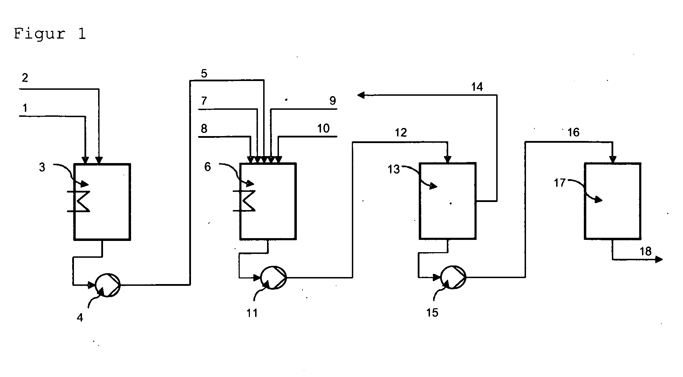

Figur 1 beschreibt eine erste Ausführungsform einer Anlage zur Durchführung des Verfahrens der vorliegenden Erfindung.

In Figur 1 ist eine erste Ausführungsform einer Anlage zur Durchführung des Verfahrens der vorliegenden Erfindung dargestellt. Diese Anlage kann beispielsweise ein, zwei oder mehr Zuführungen 1 bzw. 2, beispielsweise Leitungen oder Zuführschnecken, aufweisen, über die ein oder mehrere hyperverzweigten Polymere und/oder ein oder mehrere niedermolekulare Substanzen einem ersten Mischgefäß 3 zugeführt werden. Im Mischgefäß 3 können die zugeführten Substanzen in eine Polymerschmelze überführt werden, die mindestens ein hyperverzweigtes Polymer und mindestens eine niedermolekulare Substanz umfasst. In Mischgemäß 3 können die Komponenten fein ineinander verteilt werden. So kann beispielsweise eine Lösung, eine Dispersion'oder Suspension hergestellt werden. Vielfach bildet das hyperverzweigte Polymer die Matrixphase, in der die niedermolekulare Substanz verteilt wird. Hierfür können die zuvor beschriebenen Vorrichtungen eingesetzt werden.

Die in Mischgefäß 3 erhaltene Polymerschmelze kann beispielsweise mit einer Pumpe 4 über die Zuführung 5, beispielsweise eine Leitung, in das Mischgefäß 6 überführt werden.

Das Mischgefäß 6 kann ein, zwei, drei, vier oder mehr weitere Zuführungen 7, 8, 9, 10, beispielsweise Leitungen oder Zufuhrförderorgane, wie Schnecken oder Pumpen, aufweisen, über die beispielsweise Stabilisatoren, Emulgatoren, Warmwasser und/oder Kaltwasser zugeführt werden können. In der vorliegenden Figurenbeschreibung wird Wasser beispielhaft als zweite flüssige Phase eingesetzt. Allerdings ist dem Fachmann offensichtlich, dass jede andere zuvor als Hauptbestandteil der zweiten flüssigen Phase beschriebene Verbindung ebenfalls anstatt oder zusammen mit Wasser eingesetzt werden kann. Das Wasser dient somit lediglich als Beispiel für die zuvor dargelegten Verbindungen, das entsprechend durch die anderen Substanzen ersetzt werden kann.

Die Zuführungen 7, 8, 9, 10 können alle im Mischgefäß 6 münden. Des Weiteren können diese Zuführungen auch vor dem Eintritt in das Mischgefäß 6 zusammengeführt werden.

Vor der Zuführung der Polymerschmelze in das Mischgefäß 6 kann beispielsweise über die Zuführungen 7, 8 und 9 eine Lösung hergestellt werden, die als Hauptbestandteil beispielsweise Wasser oder Ethanol sowie Hilfsstoffe, beispielsweise Stabilisatoren und Emulgatoren umfasst. Diese Lösung kann auf eine Temperatur oberhalb der Verfestigungstemperatur des hyperverzweigten Polymers erwärmt werden. Des Weiteren können die zugeführten Komponenten bereits eine entsprechende Temperatur aufweisen.

Nach Herstellung einer entsprechenden Lösung in Mischgefäß 6 kann die in Mischgefäß 3 hergestellte Polymerschmelze dem Mischgefäß 6 zugeführt werden. In Mischgefäß 6 wird die Polymerschmelze in der zuvor beschriebenen Lösung dispergiert. Hierfür weist das Mischgefäß 6 bekannte Vorrichten zum Dispergieren auf. Hierfür können die zuvor beschriebenen Vorrichtungen eingesetzt werden.

Nach dem durch das Dispergieren die gewünschte Tropfengröße und Tropfengrößenverteilung erhalten wurde, kann die in der zweiten flüssigen Phase, beispielsweise Wasser, dispergiert vorliegende Polymerschmelze verfestigt werden. Hierzu kann beispielsweise über eine Zuführung 10 Kaltwasser in das Mischgefäß 6 eingeleitet werden. Des Weiteren kann das Mischgefäß 6 mittels'eines Kühlmediums, das durch einen Wärmeüberträger oder einen Doppelmantel geleitet wird, abgekühlt werden.

Die so erhaltenen Partikel können aus der zweiten flüssigen Phase abgetrennt werden. Hierzu kann die in Mischgefäß 6 erhaltene Zusammensetzung, die verfestigte Mikropartikel, aufweist, beispielsweise mit einer Pumpe 11 über die Leitung 12 in den Separator 13 überführt werden. Der Separator 13 dient zum Abtrennen oder Aufkonzentrieren der in der zweiten flüssigen Phase enthaltenen Mikropartikel, wobei jede der zuvor dargelegte Vorrichtung hierfür eingesetzt werden kann. Vorliegend werden im Separator die Mikropartikel von der zweiten flüssigen Phase getrennt, wobei vielfach eine Aufkonzentration ausreichend sein kann. Die abgetrennte zweite flüssige Phase, die beispielsweise Wasser, Emulgatoren und Stabilisatoren umfassen kann, kann über eine Rückführung 14, beispielsweise eine Leitung in das Mischgefäß 6 eingeleitet werden.

Die abgetrennten Mikropartikeln können beispielsweise mit einer Pumpe 15 über die Zuführung 16, beispielsweise eine Leitung, in den Trockner 17 überführt werden. Im Trockner 17 können Reste der zweiten flüssigen Phase, beispielsweise Wasser entfernt werden. Die getrockneten Mikropartikel können über die Leitung 18 dem Trockner entnommen werden.

Die Temperaturen bei der Beladung können in einem weiten Bereich liegen. Vorzugsweise wird die Beladung bei einer Temperatur im Bereich von -10° bis 150°C, besonders bevorzugt 0°C bis 80°C durchgeführt.

Die erfindungsgemäßen Präparate können einen überraschend hohen Anteil an niedermolekularer Substanz aufweisen. Gemäß einem besonderen Aspekt der vorliegenden Erfindung kann das Gewichtsverhältnis von hyperverzweigtem Polymer zur niedermolekularen Substanz vorzugsweise im Bereich von 40:1 bis 0,5:1, besonders bevorzugt im Bereich von 20:1 bis 2:1 liegen. Der Beladungsgrad kann vorzugsweise in einem Bereich von 1% bis 99%, besonders bevorzugt 5% bis 90% und ganz besonders 10 bis 30% liegen, wobei der Beladungsgrad durch den Gewichtsanteil der niedermolekularen Substanz am Gesamtgewicht des Präparats gegeben ist.

Die niedermolekularen Substanzen können aus der erfindungsgemäßen Kombination bzw. dem erfindungsgemäßen Präparat auf eine gewünschte Weise freigesetzt werden. Beispielsweise kann ein enzymatischer Abbau eingesetzt werden, um die niedermolekulare Substanz freizusetzen. Hierbei kann der Freisetzungszeitraum durch die Abbaurate gesteuert werden.

Weiterhin kann die Freisetzung über eine Änderung des pH-Wertes, Temperatur, pH, Strahlungsfrequenz und Art des Mediums gezielt gesteuert werden.

Die Art des Mediums kann beispielsweise über die Zugabe von Lösungsmittel geändert werden.

Als Lösungsmittel zur Variation des Mediums können unter anderem Wasser, Alkohole wie Ethanol oder Isopropanol komprimiertes CO₂, komprimiertes Propan, Tetrahydrofuran, Toluol, Aceton, Benzoylperoxid, wässerige HCl-Lösungen, Hexan, Essigsäure, Ethandiol, Dichlormethan, Dichlorethan oder ionische Flüssigkeiten eingesetzt werden.

Je nach Funktionalisierungsgrad des hyperverzweigten Polymers und dem Medium, in das die niedermolekulare Substanz freigesetzt werden soll, können somit die unterschiedlichsten Lösungsmittel zugegeben werden, um eine möglichst retardierte oder eine möglichst schnelle Freisetzung zu erzielen. Soll die Freisetzung der verkapselten niedermolekularen Substanzen in polaren Medien erfolgen, so ist die Freisetzung umso langsamer, je mehr OH-Gruppen des hyperverzweigten Kernpolyesters mit Fettsäuren verestert/funktionalisiert wurden. Dieser Effekt kann durch Zugabe von entsprechenden Lösungsmitteln unterstützt werden.

Weiterhin kann die Wirkstofffreisetzung insbesondere über die Dicke der Trägerpolymerhülle, die die niedermolekulare Substanz bzw. den Wirkstoff umgibt, und/oder den Funktionalisierungsgrad / Hydrophobisierungsgrad bzw. die Hydroxyzahl des hyperverzweigten Polymers gesteuert werden.

Der Freisetzungszeitraum ist umso größer, je dicker die Trägerpolymerhülle ist. Die Dicke der Trägerpolymerhülle lässt sich insbesondere durch Erhöhung der Polymerkonzentration in der Ausgangsmischung (bestehend aus mindestens einem hyperverzweigten Polymer und einer niedermolekularen Substanz) vergrößern. Hierbei kann die Art des Verkapselungsverfahrens einen Einfluss auf diese Größe haben, wobei der Fachmann durch einfache Routineversuche das für den gegebenen Zweck sinnvollste Verfahren aus den zuvor genannten auswählen kann.

Überraschenderweise wurde gefunden, dass bei hyperverzweigten Trägerpolymeren die Konzentration in der Ausgangsmischung (bestehend aus Lösungsmittel + Trägerpolymer + niedermolekularer Substanz) auch über die im Stand der Technik üblichen Polymerkonzentrationen von 10 Massenprozent bis zu einer Polymerkonzentration von 70 Massenprozent erhöht werden kann. Die letztendlich gewählte Polymerkonzentration entscheidet zusammen mit der Temperaturführung bzw. der Änderung von pH-Wert oder Lösungskraft des Lösungsmittels über die Dicke der Polymerhülle und damit über den Freisetzungszeitraum.

Neben der Dicke der Trägerpolymerhülle entscheidet der Funktionalisierungsgrad bzw. die Hydroxyzahl über den Freisetzungszeitraum. Soll die Freisetzung der verkapselten niedermolekularen Substanzen in polaren Medien erfolgen, so ist die Freisetzung umso langsamer, je mehr OH-Gruppen des hyperverzweigten Kernpolyesters mit Fettsäuren verestert/funktionalisiert wurden.

Die Präparate der vorliegenden Erfindung können vielfältig eingesetzt werden. Beispielsweise können die Präparate als Härtungsmittel, als Vernetzungsmittel, als Katalysator, als Entschäumer, als Dispergiermittel, als Additiv mit antimikrobieller oder antifungizider Wirkung, als Additiv zur Erhöhung der Kratzfestigkeit von Oberflächen, als Additiv in Beschichtungen oder Klebstoffen, als Fließverbesserer und/oder als Betonzusatzstoff eingesetzt werden. Des Weiteren können die Präparate in Kosmetika, in Arzneimitteln, in Deodorants, in Nahrungsmitteln, in Futtermitteln, in Getränken, in Wand- oder Fußbodenbelägen, in Fugenmaterialien, in Verpackungen und/oder in Lacksystemen verwendet werden.

Gemäß einem besonderen Aspekt der vorliegenden Erfindung umfasst das Präparat vorzugsweise nur geringe Mengen an Kreatin, Folsäure oder Tocopherol, falls diese Stoffe allein als niedermolekulare Substanz eingesetzt werden. Besonders bevorzugt ist die Menge an Keratin, Folsäure oder Tocopherol auf 10 Gew.-%, besonders bevorzugt 5 Gew.-% beschränkt, bezogen auf das Gewicht des Präparats, falls das Präparat keine weiteren niedermolekularen Substanzen aufweist. Präparate, die Kombinationen von Kreatin, Folsäure und/oder Tocopherol untereinander oder mit weiteren niedermolekularen Substanzen aufweisen, sind jedoch bevorzugt, wobei diese Präparate auch mehr als 10 Gew.-% an Keratin, Folsäure oder Tocopherol aufweisen können.

Nachfolgend wird die vorliegende Erfindung durch Beispiele näher erläutert, ohne dass hierdurch eine Beschränkung erfolgen soll.

### Beispiele

### Beispiel 1

Unter Verwendung der in Abbildung 1 dargestellten Anlage wurde ein erfindungsgemäßes Präparat hergestellt, welches DL-Methionin und einen hyperverzweigten Polyester umfasste.

Der eingesetzte hyperverzweigte Polyester wurde durch Hydrophobisierung eines hydrophilen hyperverzweigten Polyesters, der ein Gewichtsmittel des Molekulargewichts Mw von 3500 g/mol, eine Glastemperatur von etwa 35 °C und eine Hydroxy-Zahl von etwa 490 mg KOH/g aufwies, erhalten (kommerziell von der Firma Perstorp® unter der Bezeichnung Boltorn H30®). Die Hydrophobisierung erfolgte durch Veresterung des hydrophilen Polymers mit einer Mischung aus Eikonsäure und Behensäure (massenbezogenes Verhältnis Eikonsäure zu Behensäure = 2 zu 3), wobei 90% der Hydroxygruppen des hydrophilen Polymers umgesetzt wurden. Das Molekulargewicht M_{w} betrug 10500 g/mol. Weitere Einzelheiten zur Herstellung des hydrophilen Polymers sowie zur Veresterung können den Schriften EP-B1-0630389 oder WO 93/01760 entnommen werden.

Zur Herstellung des Präparates wurden 20 Gew.-% der Aminosäure DL-Methionin (CAS: 59-51-8; kommerziell erhältlich von der Firma Degussa® AG; gemahlen, Partikelgröße d90 im Bereich von 1 µm bis 100 µm) in dem geschmolzenen Polymer bei einer Temperatur von etwa 95°C mit einem Spiral-Rührer bei 200 Umdrehungen pro Minute in einem ersten Mischgefäß 5 Minuten dispergiert.

Im einem weiteren Mischgefäß wurde eine Mischung aus Tensiden bestehend aus 1 Gew.-% Polyvinylalkohol (M = 6000 g/mol, Polisciences®, Warrington, USA) und 0,1 Gew.-% eines ethoxylierten Fettalkohols (Tego® Alkanol L4 der Firma Degussa® AG) in Wasser bei 70 °C unter Rühren vorgelegt. Anschließend wurde die im ersten Mischgefäß hergestellte Polymerschmelze, die neben dem Polymer auch die niedermolekulare Substanz enthielt, aus dem ersten Mischgefäß unter fortwährendem Rühren mit einem ULTRA-TURRAX Rührer bei 2.000 Umdrehungen pro Minute in das zweite Mischgefäß bei 70°C überführt.

Nach einer Verweilzeit von 0,5 bis 10 Minuten und einer Abkühlung der im zweiten Mischgefäß enthaltenen Zusammensetzung auf eine Temperatur, die 15 °C unterhalb der Schmelztemperatur des Polymers lag, bildeten sich Partikel. Mit einer Schlauchpumpe wurde die Suspension einer Zentrifuge zugeleitet, in der die Wirkstoffpartikel bei 25°C von der kontinuierlichen Phase abgetrennt wurden. Anschließend wurden die Wirkstoffpartikel in einem Vakuumtrockner bei 25°C und 10 mbar für 100 h getrocknet.

Diese Partikel zeigten eine Partikelgrößenverteilung von 200 pm < d_{90,Partikel} < 300 µm. Die Partikel waren frei von unerwünschten Lösungsmitteln und bestanden aus dem hyperyerzweigten fettsäuremodifiziertem Polyester und ca. 12 Gew.-% DL-Methionin, bezogen auf die Partikelmasse.

### Beispiel 2

Das Beispiel 1 wurde im Wesentlichen wiederholt, wobei jedoch ein hyperverzweigter Polyester eingesetzt wurde, der durch Hydrophobisierung von Boltorn H30® mit einer Mischung aus Stearinsäure und Palmitinsäure (massenbezogenes Verhältnis Stearinsäure zu Palmitinsäure = 2 zu 1) erhalten wurde, wobei 50% der Hydroxygruppen des hydrophilen Polymers umgesetzt wurden. Das Molekulargewicht M_{w} betrug 7500 g/mol.

Die erhaltenen Partikel zeigten eine Partikelgrößenverteilung von 150 µm < d_{90,Partikel} < 300 µm. Die Partikel waren frei von unerwünschten Lösungsmitteln und bestanden aus dem hyperverzweigten fettsäuremodifiziertem Polyester und ca. 16 Gew.-% DL-Methionin, bezogen auf die Partikelmasse.

Die Lagerstabilität, die Scherstabilität und die enzymatische Abbaubarkeit der erhaltenen Mikropartikel wurden untersucht.

Die Bestimmung der Lagerstabilität erfolgte durch Lagerung der trocknen Mikropartikel in einem Glasgefäß, wobei die Lagerung bei Raumtemperatur in sauerstoffhaltiger Atmosphäre (Luft) erfolgte. Nach einer Lagerzeit von sechs Monaten betrug der chromatographisch mittels HPLC bestimmte Methioningehalt mindestens 98% des ursprünglichen Wertes.

Zur Untersuchung der Scherstabilität wurde eine Dispersion der erhaltenen Mikropartikeln in einem Pharmaöl (Paraffin Oil WINOG 100 Pharma von Univar GmbH) hergestellt. Hierzu wurden die Partikel in ein Standardpharmaöl (Paraffin Oil WINOG 100 Pharma von Firma Univar® GmbH) mit einem ULTRA-TURRAX Rührer, der für 1 Minute mit 20000 Umdrehungen pro Minute rührte, eingearbeitet. Ein Vergleich der Mikroskopaufnahmen der Wirkstoffpartikel vor und nach dem Einarbeiten in das Standardpharmaöl zeigte, dass keine Veränderung der Partikelintegrität zu sehen ist und somit die Schutzwirkung für den biologisch aktiven Wirkstoff DL-Methionin aufrecht erhalten werden konnte.

Die enzymatische Freisetzung des in den Mikropartikeln enthaltenen Wirkstoffs erfolgte mit einer Lipase. Hierzu wurden 0.22 g Mikropartikel in 15 ml Lösung des Enzyms Lipomod 34P (Biocatalyst Lmt., UK) in Phosphatpuffer (pH 5.01) (die Konzentration des Lipomods 34P betrug 0.5 mg/ml) suspendiert. Die Probe wurde in einem Wasserbad bei 37°C ohne Durchmischung gelagert. In regelmäßigen Zeitabständen wurde eine Probe von 2 ml entnommen. Die Konzentration des Wirkstoffs wurde chromatographisch mittels HPLC analysiert. Nach 24 Stunden wurden ca. 70 % des verkapselten Methionins freigesetztet. Dahingegen wurden in die enzymfreie Pufferlösung weniger als 20 % des verkapselten Methionins nach 24 Stunden freigesetzt.

### Beispiel 3

Das Beispiel 2 wurde im Wesentlichen wiederholt, wobei jedoch L-Leucin (CAS: 61-90-5; kommerziell erhältlich von der Firma Degussa® AG; Partikelgröße d90 im Bereich von 1 µm bis 10 µm) anstatt von DL-Methionin eingesetzt wurde. Hierbei wurde die Polymerschmelze bei einer Temperatur von etwa 85°C hergestellt. Des Weiteren wurde der ULTRA-TURRAX Rührer bei der Überführung der Polymerschmelze in die zweite flüssige Phase mit 3.000 Umdrehungen pro Minute betrieben.

Die erhaltenen Partikel zeigten eine Partikelgrößenverteilung von 10 µm < d₉₀,_{Partikel} < 50 µm. Die Partikel waren frei von unerwünschten Lösungsmitteln und bestanden aus dem hyperverzweigten fettsäuremodifiziertem Polyester und ca. 16 Gew.-% L-Leucin, bezogen auf die Partikelmasse.

Bei der Scherstabilitätsuntersuchtung zeigten diese Mikropartikel ebenfalls eine hohe Stabilität. Ein Vergleich der Mikroskopaufnahmen der Wirkstoffpartikel vor und nach dem Einarbeiten in das Standardpharmaöl zeigte, dass keine Veränderung der Partikelintegrität zu sehen ist und somit die Schutzwirkung für den biologisch aktiven Wirkstoff L-Leucin aufrecht erhalten werden konnte.

### Beispiel 4

Das Beispiel 2 wurde im Wesentlichen wiederholt, wobei jedoch L-Lysin (CAS: 56-87-1; gemahlen; 50 Gew.-%; kommerziell erhältlich von der Firma Degussa® AG; Partikelgröße d90 im Bereich von 1 µm bis 10 µm) anstatt von DL-Methionin eingesetzt wurde. Hierbei wurde die Polymerschmelze bei einer Temperatur von etwa 85°C hergestellt.

Die erhaltenen Partikel zeigten eine Partikelgrößenverteilung von 150 pm < d₉₀,_{Partikel}< 300 µm. Die Partikel waren frei von unerwünschten Lösungsmitteln und bestanden aus dem hyperverzweigten fettsäuremodifiziertem Polyester und ca. 8 Gew.-% L- Lysin, bezogen auf die Partikelmasse.

Bei der Scherstabilitätsuntersuchtung zeigten diese Mikropartikel ebenfalls eine hohe Stabilität. Ein Vergleich der Mikroskopaufnahmen der Wirkstoffpartikel vor und nach dem Einarbeiten in das Standardpharmaöl zeigte, dass keine Veränderung der Partikelintegrität zu sehen ist und somit die Schutzwirkung für den biologisch aktiven Wirkstoff L-Lysin aufrecht erhalten werden konnte.

### Beispiel 5

Das Beispiel 1 wurde im Wesentlichen wiederholt, wobei jedoch ein hyperverzweigter Polyester eingesetzt wurde, der durch Hydrophobisierung von Boltorn H30® mit einer Mischung aus Stearinsäure und Palmitinsäure (massenbezogenes Verhältnis Stearinsäure zu Palmitinsäure = 2 zu 1) erhalten wurde, wobei 95% der Hydroxygruppen des hydrophilen Polymers umgesetzt wurden. Das Molekulargewicht M_{w} betrug 10000 g/mol. Darüber hinaus wurde L-Tryptophan (CAS: 73-22-3; kommerziell erhältlich von der Firma Degussa® AG; Partikelgröße d90 im Bereich von 1 µm bis 10 µm) anstatt von DL-Methionin als niedermolekulare Substanz eingesetzt. Hierbei wurde die Polymerschmelze bei einer Temperatur von etwa 85°C hergestellt. Des Weiteren wurde der ULTRA-TURRAX Rührer bei der Überführung der Polymerschmelze in die zweite flüssige Phase mit 3.000 Umdrehungen pro Minute betrieben.

Die erhaltenen Partikel zeigten eine Partikelgrößenverteilung von 10 µm < d₉₀,_{Partikel} < 60 µm. Die Partikel waren frei von unerwünschten Lösungsmitteln und bestanden aus dem hyperverzweigten fettsäuremodifiziertem Polyester und ca. 16 Gew.-% L-Tryptophan, bezogen auf die Partikelmasse.

Bei der Scherstabilitätsuntersuchtung zeigten diese Mikropartikel ebenfalls eine hohe Stabilität. Ein Vergleich der Mikroskopaufnahmen der Wirkstoffpartikel vor und nach dem Einarbeiten in das Standardpharmaöl zeigte, dass keine Veränderung der Partikelintegrität zu sehen ist und somit die Schutzwirkung für den biologisch aktiven Wirkstoff L-Tryptophan aufrecht erhalten werden konnte.

### Beispiel 6

Das Beispiel 5 wurde im Wesentlichen wiederholt, wobei jedoch eine Bananenextraktlösung (20 Gew.-% Bananenextrakt in Ethanol) anstatt von L-Tryptophan eingesetzt wurde. Hierbei wurden wobei 20 Vol.-% Bananenextraktlösung in dem geschmolzenen Polymer gelöst.

Die erhaltenen Partikel zeigten eine Partikelgrößenverteilung von 10 µm < d₉₀,_{Partikel} < 60 µm. Die Partikel waren frei von unerwünschten Lösungsmitteln und bestanden aus dem hyperverzweigten fettsäuremodifiziertem Polyester und ca. 3 Gew.-% Bananenextrakt, bezogen auf die Partikelmasse.

### Beispiel 7

Das Beispiel 5 wurde im Wesentlichen wiederholt, wobei jedoch eine Kombination von Erdbeerextraktlösung (20 Gew.-% Erdbeerextrakt in Ethanol) und Kreatin (Creapure, CAS: 50-00-1, gemahlen, kommerziell erhältlich von der Firma Degussa® AG; Partikelgröße d90 im Bereich von 0 µm bis 10 µm) anstatt von L-Tryptophan eingesetzt wurde. Hierbei wurden 5 Vol.-% Erdbeerextraktlösung und 20 Gew.-% Kreatin in dem geschmolzenen Polymer gelöst.

Die erhaltenen Partikel zeigten eine Partikelgrößenverteilung von 10 µm < d₉₀,_{Partikel} < 60 µm. Die Partikel waren frei von unerwünschten Lösungsmitteln und bestanden aus dem hyperverzweigten fettsäuremodifiziertem Polyester und ca. 0,8 Gew.-% Erdbeerextrakt und 16 Gew.-% Kreatin, bezogen auf die Partikelmasse.

### Beispiel 8

Das Beispiel 5 wurde im Wesentlichen wiederholt, wobei jedoch eine Kombination von L(+)-Ascorbinsäure (Vitamin C, CAS: 50-81-7, gemahlen, kommerziell erhältlich von Firma AcrosOrganics®, Partikelgröße d90 im Bereich von 0 µm bis 10 µm) und Kreatin (Creapure, CAS: 50-00-1, gemahlen, kommerziell erhältlich von der Firma Degussa® AG; Partikelgröße d90 im Bereich von 0 µm bis 10 µm) anstatt von L-Tryptophan eingesetzt wurde. Hierbei wurden 10 Gew.-% L(+)-Ascorbinsäure und 10 Gew.-% Kreatin in dem geschmolzenen Polymer dispergiert.

Die erhaltenen Partikel zeigten eine Partikelgrößenverteilung von 10 µm < d₉₀,_{Partikel} < 50 µm. Die Partikel waren frei von unerwünschten Lösungsmitteln und bestanden aus dem hyperverzweigten fettsäuremodifiziertem Polyester und ca. 8 Gew.-% L(+)-Ascorbinsäure und 8 Gew.-% Kreatin, bezogen auf die Partikelmasse.

### Beispiel 9

Das Beispiel 5 wurde im Wesentlichen wiederholt, wobei jedoch eine Kombination von Zitronenaromalösung (20 Gew.-% Zitronenaroma in Ethanol) und Percarbonat (Natriumpercarbonat für Waschmittel, kommerziell erhältlich von der AcrosOrganics®; Partikelgröße d90 im Bereich von 1 µm bis 10 µm) Partikelgröße d90 im Bereich von 0 µm bis 10 µm) anstatt von L-Tryptophan eingesetzt wurde. Hierbei wurden 5 Vol.-% Zitronenaromalösung und 20 Gew.-% Natriumpercarbonat in dem geschmolzenen Polymer gelöst. Des Weiteren wurde der ULTRA-TURRAX Rührer bei der Überführung der Polymerschmelze in die zweite flüssige Phase mit 2.000 Umdrehungen pro Minute betrieben.

Die erhaltenen Partikel zeigten eine Partikelgrößenverteilung von 10 µm < d₉₀,_{Partikel} < 60 µm. Die Partikel waren frei von unerwünschten Lösungsmitteln und bestanden aus dem hyperverzweigten fettsäuremodifiziertem Polyester und ca. 0,8 Gew.-% Zitronenaroma und 16 Gew.-% Natriumpercarbonat, bezogen auf die Partikelmasse.

### Beispiel 10

Das Beispiel 2 wurde im Wesentlichen wiederholt, wobei jedoch Ammoniumpersulfat (CAS: 7727-54-0; kommerziell erhältlich von der Firma Degussa® AG; Partikelgröße d90 im Bereich von 1 µm bis 200 µm) anstatt von DL-Methionin eingesetzt wurde. Hierbei wurde die Polymerschmelze bei einer Temperatur von etwa 70°C hergestellt.

Die erhaltenen Partikel zeigten eine Partikelgrößenverteilung von 250 µm < d_{90,Partikel} < 400 µm. Die Partikel waren frei von unerwünschten Lösungsmitteln und bestanden aus dem hyperverzweigten fettsäuremodifiziertem Polyester und ca. 16 Gew.-% Ammoniumpersulfat, bezogen auf die Partikelmasse.

### Beispiel 11

Das Beispiel 2 wurde im Wesentlichen wiederholt, wobei jedoch L-Tryptophan (CAS: 73-22-3; kommerziell erhältlich von der Firma Degussa® AG; Partikelgröße d90 im Bereich von 1 µm bis 10 µm) anstatt von DL-Methionin eingesetzt wurde. Hierbei wurde die Polymerschmelze bei einer Temperatur von etwa 85°C hergestellt. Des Weiteren wurde der ULTRA-TURRAX Rührer bei der Überführung der Polymerschmelze in die zweite flüssige Phase mit 1.000 Umdrehungen pro Minute betrieben.

Die erhaltenen Partikel zeigten eine Partikelgrößenverteilung von 300 µm < d₉₀,_{Partikel} < 500 µm. Die Partikel waren frei von unerwünschten Lösungsmitteln und bestanden aus dem hyperverzweigten fettsäuremodifiziertem Polyester und ca. 16 Gew.-% L-Tryptophan, bezogen auf die Partikelmasse. Dieses Beispiel zeigt, dass die Partikelgröße durch das erfindungsgemäße Verfahren leicht auf einen gewünschten Wert eingestellt werden kann, wobei große Partikel beispielsweise keine Staubbildung zeigen.

## Patentansprüche

1. Partikelförmiges Präparat umfassend mindestens eine niedermolekulare Substanz mit einer Molmasse im Bereich von 15 g/mol bis 1000 g/mol und mindestens ein hyperverzweigtes Polymer, **dadurch gekennzeichnet, dass** die Partikel eine Größe im Bereich von 1 µm bis 1000 µm aufweisen, die niedermolekulare Substanz in einer Hülle eingebettet ist, die hyperverzweigtes Polymer umfasst, und das hyperverzweigte Polymer einen hydrophilen Kern mit Polyestereinheiten sowie hydrophobe Endgruppen umfasst, wobei das hyperverzweigte Polymer ein Molekulargewicht größer oder gleich 6000 g/mol und eine Hydroxyzahl im Bereich von 0 bis 200 mg KOH/g aufweist, der Verzweigungsgrad im Bereich von 20 bis 70% liegt und das hyperverzweigte Polymer eine Schmelztemperatur von mindestens 30°C aufweist.

2. Präparat gemäß Anspruch 1, **dadurch gekennzeichnet, dass** das hyperverzweigte Polymer einen Funktionalisierungsgrad von mindestens 30% aufweist.

3. Präparat gemäß Anspruch 1 oder 2, **dadurch gekennzeichnet, dass** das hyperverzweigte Polymer eine Wasserlöslichkeit nach der Kolbenmethode bei 40°C von höchstens 10 Massenprozent aufweist.

4. Präparat gemäß mindestens einem der vorhergehenden Ansprüche, **dadurch gekennzeichnet, dass** der hydrophile Kern mindestens 90 Gew.-% an Wiederholungseinheiten aufweist, die von Polyester bildenden Monomeren abgeleitet sind.

5. Präparat gemäß mindestens einem der vorhergehenden Ansprüche, **dadurch gekennzeichnet, dass** der hydrophile Kern eine zentrale Einheit, die von einem Initiatormolekül mit mindestens zwei Hydoxygruppen abgeleitet ist, und Wiederholungseinheiten, die von Monomeren mit mindestens einer Carboxylgruppe und mindestens zwei Hydroxygruppen abgeleitet sind, aufweist.

6. Präparat gemäß Anspruch 5, **dadurch gekennzeichnet, dass** das Initiatormolekül ein aliphatisches Polyol ist.

7. Präparat gemäß Anspruch 5 oder 6, **dadurch gekennzeichnet, dass** das Initiatormolekül ausgewählt ist aus Ditrimethylolpropan, Ditrimethylolethan, Dipentaerythrit, Pentaerythrit, alkoxliertem Pentaerythrit, Trimethylolethan, Trimethylolpropan, alkoxyliertem Trimethylolpropan, Glycerin, Neopentylalkohol, Dimethylolpropan und/oder 1,3-Dioxan-5,5-dimethanol.

8. Präparat gemäß mindestens einem der Ansprüche 5 bis 7, **dadurch gekennzeichnet, dass** die Monomeren ausgewählt sind aus der Gruppe bestehend aus Dimethylpropionsäure, α,α-Bis(hydroxymethyl)buttersäure, α,α,α,-Tris(hydroxymethyl)essigsäure, α,α-Bis(hydroxymethyl)-valeriansäure, α,α-Bis(hydroxy)propionsäure und/oder 3,5-Dihydroxybenzoesäure.

9. Präparat gemäß mindestens einem der vorhergehenden Ansprüche, **dadurch gekennzeichnet, dass** die hydrophoben Endgruppen durch Gruppen gebildet werden, die von Carbonsäuren mit mindestens 10 Kohlenstoffatomen abgeleitet sind.

10. Präparate gemäß Anspruch 9, **dadurch gekennzeichnet, dass** die Carbonsäuren einen Schmelzpunkt von mindestens 40°C aufweisen.

11. Präparat gemäß Anspruch 9 oder 10, **dadurch gekennzeichnet, dass** mindestens ein Teil der Carbonsäuren ausgewählt ist aus Decansäure, Dodecansäure, Tetradecansäure, Hexadecansäure, Heptadecansäure, Octadecansäure, Eicosansäure, Docosansäure und Tetracosansäure.

12. Präparat gemäß mindestens einem der vorhergehenden Ansprüche 9 bis 11, **dadurch gekennzeichnet, dass** mindestens ein Teil der hydrophoben Endgruppen durch Gruppen gebildet werden, die von Carbonsäuren mit höchstens 18 Kohlenstoffatomen abgeleitet sind.

13. Präparat gemäß mindestens einem der vorhergehenden Ansprüche 9 bis 11, **dadurch gekennzeichnet, dass** der Anteil der Carbonsäuren mit 12 bis 18 Kohlenstoffatomen mindestens 30 Gew.-%, bezogen auf das Gewicht der zur Hydrophobisierung eingesetzten Carbonsäuren, beträgt.

14. Präparat gemäß mindestens einem der vorhergehenden Ansprüche, **dadurch gekennzeichnet, dass** das hyperverzweigte Polymer eine Säurezahl im Bereich von 0 bis 20 mg KOH/g aufweist.

15. Präparat gemäß mindestens einem der vorhergehenden Ansprüche, **dadurch gekennzeichnet, dass** das hyperverzweigte Polymer eine Schmelztemperatur von höchstens 57°C aufweist.

16. Präparat gemäß mindestens einem der vorhergehenden Ansprüche, **dadurch gekennzeichnet, dass** der hydrophile Kern eine vor der Hydrophobisierung gemessene Hydroxyzahl im Bereich von 400 bis 600 mg KOH/g aufweist.

17. Präparat gemäß mindestens einem der vorhergehenden Ansprüche, **dadurch gekennzeichnet, dass** die niedermolekulare Substanz ein Verbindung mit einer Peroxidgruppe ist.

18. Präparat gemäß mindestens einem der vorhergehenden Ansprüche, **dadurch gekennzeichnet, dass** die niedermolekulare Substanz eine Aminosäure ist.

19. Präparat gemäß mindestens einem der vorhergehenden Ansprüche, **dadurch gekennzeichnet, dass** die niedermolekulare Substanz ein Katalysator ist.

20. Präparat gemäß mindestens einem der vorhergehenden Ansprüche, **dadurch gekennzeichnet, dass** die niedermolekulare Substanz ein Farbstoff und/oder Pigment ist.

21. Präparat gemäß mindestens einem der vorhergehenden Ansprüche, **dadurch gekennzeichnet, dass** die niedermolekulare Substanz ein Vitamin ist.

22. Präparat gemäß mindestens einem der vorhergehenden Ansprüche, **dadurch gekennzeichnet, dass** die niedermolekulare Substanz ein Monomer ist.

23. Präparat gemäß mindestens einem der vorhergehenden Ansprüche, **dadurch gekennzeichnet, dass** die niedermolekulare Substanz ein Geschmacks- oder Aromastoff ist.

24. Präparat gemäß mindestens einem der vorhergehenden Ansprüche, **dadurch gekennzeichnet, dass** die niedermolekulare Substanz eine biologisch aktive Komponente, insbesondere ein Arzneimittel, ein Vitamin, ein Enzym, ein Coenzym, und/oder ein Pflanzenextrakt ist.

25. Präparat gemäß mindestens einem der vorhergehenden Ansprüche, **dadurch gekennzeichnet, dass** die niedermolekulare Substanz ein Initiator, ein Silikon, ein Tensid, eine Kieselsäure, ein Silan, ein Lösungsmittel, ein Füllstoff, ein Reaktivvernetzer, ein Detergenz, eine Haarfarbe und/oder ein Betonzusatzmittel ist.

26. Präparat gemäß mindestens einem der vorhergehenden Ansprüche, **dadurch gekennzeichnet, dass** das hyperverzweigte Polymer enzymatisch abbaubar ist.

27. Präparat gemäß mindestens einem der vorhergehenden Ansprüche, **dadurch gekennzeichnet, dass** mindestens 80 Gew.-% der Partikel innerhalb eines Größenbereichs von 100 µm oder weniger liegen.

28. Präparat gemäß mindestens einem der vorhergehenden Ansprüche, **dadurch gekennzeichnet, dass** das hyperverzweigte Polymer bei 48°C in einer 50 Ges.-%/50 Gew.-% Mischung mit Wasser lediglich Flüssig-Flüssig-Gleichgewichte, jedoch keine Fest-Flüssig-Gleichgewichte Fest-Flüssig-Flüssig-Gleichgewichte aufweist.

29. Verfahren zur Herstellung von Präparaten gemäß mindestens einem der Ansprüche 1 bis 28 durch Mischen eines hyperverzweigten Polymers und mindestens einer niedermolekularen Substanz.

30. Verwendung eines Präparats gemäß mindestens einem der Ansprüche 1 bis 28 als Härtungsmittel, als Vernetzungsmittel, als Katalysator, als Entschäumer, als Dispergiermittel, als Additiv mit antimikrobieller oder antifungizider Wirkung, als Additiv zur Erhöhung der Kratzfestigkeit von Oberflächen, als Additiv in Beschichtungen oder Klebstoffen, als Fließverbesserer und/oder als Betonzusatzstoff.

31. Verwendung eines Präparats gemäß mindestens einem der Ansprüche 1 bis 28 in Kosmetika, in Arzneimitteln, in Deodorants, in Nahrungsmitteln, in Futtermitteln, in Getränken, in Wand- oder Fußbodenbelägen, in Fugenmaterialien, in Verpackungen und/oder in Lacksystemen.

32. Verfahren zur Freisetzung von niedermolekularen Substanzen aus Präparaten gemäß mindestens einem der Ansprüche 1 bis 28, **dadurch gekennzeichnet, dass** das hyperverzweigte Polymer enzymatisch abgebaut wird.

33. Verfahren zur Herstellung von Präparaten gemäß mindestens einem der Ansprüche 1 bis 28, **dadurch gekennzeichnet, dass** ein komprimiertes Gas verwendet wird oder mindestens zwei flüssige Phasen auftreten, wobei beide Phasen die niedermolekulare Substanz enthalten und mindestens eine Flüssigphase ein hyperverzweigtes Polymer enthält.

34. Verfahren zur Herstellung von Präparaten gemäß mindestens einem der Ansprüche 1 bis 28, umfassend die Schritte Herstellen einer Polymerschmelze umfassend mindestens ein hyperverzweigtes Polymer und mindestens eine niedermolekulare Substanz, Einleiten der Polymerschmelze in eine zweite flüssige Phase, in der das hyperverzweigte Polymer schwer löslich ist und die eine Verfestigungstemperatur unterhalb der Verfestigungstemperatur des hyperverzweigten Polymers aufweist, Dispergieren der Polymerschmelze in der zweiten flüssigen Phase bei einer Temperatur, die größer oder gleich der Verfestigungstemperatur des hyperverzweigten Polymers ist und Verfestigen der in der zweiten flüssigen Phase dispergierten Polymerschmelze.

## Claims

1. Particulate preparation comprising at least one low molecular weight substance having a molar mass in the range from 15 g/mol to 1000 g/mol and at least one hyperbranched polymer, **characterized in that** the particles have a size in the range from 1 µm to 1000 µm, the low molecular weight substance is embedded in a shell which comprises hyperbranched polymer, and the hyperbranched polymer comprises a hydrophilic core having polyester units and hydrophobic end groups, said hyperbranched polymer having a molecular weight greater than or equal to 6000 g/mol and a hydroxyl number in the range from 0 to 200 mg KOH/g, the degree of branching being in the range from 20 to 70%, and said hyperbranched polymer having a melting point of at least 30°C.

2. Preparation according to Claim 1, **characterized in that** said hyperbranched polymer has a degree of functionalization of at least 30%.

3. Preparation according to Claim 1 or 2, **characterized in that** said hyperbranched polymer has a water solubility by the flask method at 40°C of at most 10% by mass.

4. Preparation according to at least one of the preceding claims, **characterized in that** said hydrophilic core has at least 90% by weight of repeat units derived from polyester-forming monomers.

5. Preparation according to at least one of the preceding claims, **characterized in that** said hydrophilic core has a central unit derived from an initiator molecule having at least two hydroxyl groups, and repeat units derived from monomers having at least one carboxyl group and at least two hydroxyl groups.

6. Preparation according to Claim 5, **characterized in that** the initiator molecule is an aliphatic polyol.

7. Preparation according to Claim 5 or 6, **characterized in that** said initiator molecule is selected from ditrimethylolpropane, ditrimethylolethane, dipentaerythritol, pentaerythritol, alkoxylated pentaerythritol, trimethylolethane, trimethylolpropane, alkoxylated trimethylolpropane, glycerol, neopentyl alcohol, dimethylolpropane and/or 1,3-dioxane-5,5-dimethanol.

8. Preparation according to at least one of Claims 5 to 7, **characterized in that** said monomers are selected from the group consisting of dimethylpropionic acid, α,α-bis(hydroxymethyl)butyric acid, α,α,α-tris(hydroxymethyl)acetic acid, α,α-bis(hydroxymethyl)valeric acid, α,α-bis(hydroxy)propionic acid, and/or 3,5-dihydroxybenzoic acid.

9. Preparation according to at least one of the preceding claims, **characterized in that** said hydrophobic end groups are formed by groups derived from carboxylic acids having at least 10 carbon atoms.

10. Preparation according to Claim 9, **characterized in that** said carboxylic acids have a melting point of at least 40°C.

11. Preparation according to Claim 9 or 10, **characterized in that** at least some of said carboxylic acids are selected from decanoic acid, dodecanoic acid, tetradecanoic acid, hexadecanoic acid, heptadecanoic acid, octadecanoic acid, eicosanoic acid, docosanoic acid and tetracosanoic acid.

12. Preparation according to at least one of the preceding Claims 9 to 11, **characterized in that** at least some of said hydrophobic end groups are formed by groups derived from carboxylic acids having at most 18 carbon atoms.

13. Preparation according to at least one of the preceding Claims 9 to 11, **characterized in that** the proportion of said carboxylic acids having from 12 to 18 carbon atoms is at least 30% by weight, based on the weight of the carboxylic acids used for hydrophobization.

14. Preparation according to at least one of the preceding claims, **characterized in that** said hyperbranched polymer has an acid number in the range from 0 to 20 mg KOH/g.

15. Preparation according to at least one of the preceding claims, **characterized in that** said hyperbranched polymer has a melting point of at most 57°C.

16. Preparation according to at least one of the preceding claims, **characterized in that** said hydrophilic core has a hydroxyl number, measured before the hydrophobization, in the range from 400 to 600 mg KOH/g.

17. Preparation according to at least one of the preceding claims, **characterized in that** said low molecular weight substance is a compound having a peroxide group.

18. Preparation according to at least one of the preceding claims, **characterized in that** said low molecular weight substance is an amino acid.

19. Preparation according to at least one of the preceding claims, **characterized in that** said low molecular weight substance is a catalyst.

20. Preparation according to at least one of the preceding claims, **characterized in that** said low molecular weight substance is a dye and/or pigment.

21. Preparation according to at least one of the preceding claims, **characterized in that** said low molecular weight substance is a vitamin.

22. Preparation according to at least one of the preceding claims, **characterized in that** said low molecular weight substance is a monomer.

23. Preparation according to at least one of the preceding claims, **characterized in that** said low molecular weight substance is a flavoring or aroma.

24. Preparation according to at least one of the preceding claims, **characterized in that** said low molecular weight substance is a biologically active component, especially a medicament, a vitamin, an enzyme, a coenzyme and/or a plant extract.

25. Preparation according to at least one of the preceding claims, **characterized in that** said low molecular weight substance is an initiator, a silicone, a surfactant, a silica, a silane, a solvent, a filler, a reactive crosslinker, a detergent, a hair dye and/or a concrete additive.

26. Preparation according to at least one of the preceding claims, **characterized in that** said hyperbranched polymer is enzymatically degradable.

27. Preparation according to at least one of the preceding claims, **characterized in that** at least 80% by weight of said particles are within a size range of 100 µm or less.

28. Preparation according to at least one of the preceding claims, **characterized in that** the hyperbranched polymer, at 48°C in a 50% by weight/50% by weight mixture with water, has only liquid-liquid equilibria, but no solid-liquid equilibria, solid-liquid-liquid equilibria.

29. Process for producing preparations according to at least one of Claims 1 to 28 by mixing a hyperbranched polymer and at least one low molecular weight substance.

30. Use of a preparation according to at least one of Claims 1 to 28 as a hardener, as a crosslinker, as a catalyst, as a defoamer, as a dispersant, as an additive with antimicrobial or antifungicidal action, as an additive for increasing the scratch resistance of surfaces, as an additive in coatings or adhesives, as a flow improver and/or as a concrete additive.

31. Use of a preparation according to at least one of Claims 1 to 28 in cosmetics, in medicaments, in deodorants, in foods, in animal feeds, in drinks, in wall or floor coverings, in joint materials, in packaging and/or in paint systems.

32. Process for releasing low molecular weight substances from preparations according to at least one of Claims 1 to 28, **characterized in that** the hyperbranched polymer is enzymatically degraded.

33. Process for producing preparations according to at least one of Claims 1 to 28, **characterized in that** a compressed gas is used or at least two liquid phases occur, in which case the two said phases comprise the low molecular weight substance and at least one liquid phase comprises a hyperbranched polymer.

34. Process for producing preparations according to at least one of Claims 1 to 28, comprising the steps of preparing a polymer melt comprising at least one hyperbranched polymer and at least one low molecular weight substance, introducing said polymer melt into a second liquid phase in which said hyperbranched polymer is sparingly soluble and which has a solidification temperature below the solidification temperature of said hyperbranched polymer, dispersing said polymer melt in said second liquid phase at a temperature which is greater than or equal to the solidification temperature of said hyperbranched polymer, and solidifying said polymer melt dispersed in said second liquid phase.

## Revendications

1. Préparation particulaire comprenant au moins une substance de faible masse moléculaire, ayant une masse moléculaire dans la plage de 15 g/mole à 1 000 g/mole, et au moins un polymère hyper-ramifié, **caractérisée en ce que** les particules ont une taille dans la plage de 1 µm à 1 000 µm, la substance de faible masse moléculaire est incluse dans une enveloppe qui comprend un polymère hyper-ramifié, et le polymère hyper-ramifié comprend un noyau hydrophile à motifs polyester ainsi que des groupes terminaux hydrophobes, le polymère hyper-ramifié ayant une masse moléculaire supérieure ou égale à 6 000 g/mole et un indice de groupes hydroxy dans la plage de 0 à 200 mg de KOH/g, le degré de ramification se situant dans la plage de 20 à 70 % et le polymère hyper-ramifié présentant une température de fusion d'au moins 30 °C.

2. Préparation selon la revendication 1, **caractérisée en ce que** le polymère hyper-ramifié présente un degré de fonctionnalisation d'au moins 30 %.

3. Préparation selon la revendication 1 ou 2, **caractérisée en ce que** le polymère hyper-ramifié présente une solubilité dans l'eau selon la méthode en flacon, à 40 °C, d'au moins 10 % en masse.

4. Préparation selon au moins l'une des revendications précédentes, **caractérisée en ce que** le noyau hydrophile comporte au moins 90 % en poids de motifs répétitifs qui sont dérivés de monomères constituant le polyester.

5. Préparation selon au moins l'une des revendications précédentes, **caractérisée en ce que** le noyau hydrophile comporte un motif central qui est dérivé d'une molécule d'amorceur comportant au moins deux groupes hydroxy, et des motifs répétitifs qui sont dérivés de monomères comportant au moins un groupe carboxy et au moins deux groupes hydroxy.

6. Préparation selon la revendication 5, **caractérisée en ce que** la molécule d'amorceur est un polyol aliphatique.

7. Préparation selon la revendication 5 ou 6, **caractérisée en ce que** la molécule d'amorceur est choisie parmi le ditriméthylolpropane, le ditriméthyloléthane, le dipentaérythritol, le pentaérythritol, un pentaérythritol alcoxylé, le triméthyloléthane, le triméthylolpropane, un triméthylolpropane alcoxylé, le glycérol, l'alcool néopentylique, le diméthylolpropane et/ou le 1,3-dioxanne-5,5-diméthanol.

8. Préparation selon au moins l'une des revendications 5 à 7, **caractérisée en ce que** les monomères sont choisis dans le groupe constitué par l'acide diméthylpropionique, l'acide α,α-bis-(hydroxyméthyl)butyrique, l'acide α,α,a-tris-(hydroxyméthyl)acétique, l'acide a,a-bis(hydroxy-méthyl)-valérique, l'acide α,α-bis(hydroxy)propionique et/ou l'acide 3,5-dihydroxybenzoïque.

9. Préparation selon au moins l'une des revendications précédentes, **caractérisée en ce que** les groupes terminaux hydrophobes sont constitués de groupes qui sont dérivés d'acides carboxyliques ayant au moins 10 atomes de carbone.

10. Préparation selon la revendication 9, **caractérisée en ce que** les acides carboxyliques ont un point de fusion d'au moins 40 °C.

11. Préparation selon la revendication 9 ou 10, **caractérisée en ce qu'**au moins une partie des acides carboxyliques est choisie parmi l'acide décanoïque, l'acide dodécanoïque, l'acide tétradécanoïque, l'acide hexadécanoïque, l'acide heptadécanoïque, l'acide octadécanoïque, l'acide eicosanoïque, l'acide docosanoïque et l'acide tétracosanoïque.

12. Préparation selon au moins l'une des revendications 9 à 11 précédentes, **caractérisée en ce qu'**au moins une partie des groupes terminaux hydrophobes sont constitués de groupes qui sont dérivés d'acides carboxyliques ayant au maximum 18 atomes de carbone.

13. Préparation selon au moins l'une des revendications 9 à 11 précédentes, **caractérisée en ce que** la concentration des acides carboxyliques ayant de 12 à 18 atomes de carbone est d'au moins 30 % en poids, par rapport au poids des acides carboxyliques utilisés pour l'hydrophobisation.

14. Préparation selon au moins l'une des revendications précédentes, **caractérisée en ce que** le polymère hyper-ramifié présente un indice d'acide dans la plage de 0 à 20 mg de KOH/g.

15. Préparation selon au moins l'une des revendications précédentes, **caractérisée en ce que** le polymère hyper-ramifié a une température de fusion d'au maximum 57 °C.

16. Préparation selon au moins l'une des revendications précédentes, **caractérisée en ce que** le noyau hydrophile présente un indice de groupes hydroxy, mesuré avant l'hydrophobisation, dans la plage de 400 à 600 mg de KOH/g.

17. Préparation selon au moins l'une des revendications précédentes, **caractérisée en ce que** la substance de faible masse moléculaire est un composé comportant un groupe peroxyde.

18. Préparation selon au moins l'une des revendications précédentes, **caractérisée en ce que** la substance de faible masse moléculaire est un acide aminé.

19. Préparation selon au moins l'une des revendications précédentes, **caractérisée en ce que** la substance de faible masse moléculaire est un catalyseur.

20. Préparation selon au moins l'une des revendications précédentes, **caractérisée en ce que** la substance de faible masse moléculaire est un colorant et/ou un pigment.

21. Préparation selon au moins l'une des revendications précédentes, **caractérisée en ce que** la substance de faible masse moléculaire est une vitamine.

22. Préparation selon au moins l'une des revendications précédentes, **caractérisée en ce que** la substance de faible masse moléculaire est un monomère.

23. Préparation selon au moins l'une des revendications précédentes, **caractérisée en ce que** la substance de faible masse moléculaire est un agent de sapidité ou un arôme.

24. Préparation selon au moins l'une des revendications précédentes, **caractérisée en ce que** la substance de faible masse moléculaire est un composant biologiquement actif, en particulier un médicament, une vitamine, une enzyme, une co-enzyme, et/ou un extrait de plante.

25. Préparation selon au moins l'une des revendications précédentes, **caractérisée en ce que** la substance de faible masse moléculaire est un amorceur, un silicone, un tensioactif, un acide silicique, un silane, un solvant, une charge, un agent de réticulation réactif, un détergent, une couleur pour cheveux et/ou un additif pour béton.

26. Préparation selon au moins l'une des revendications précédentes, **caractérisée en ce que** le polymère hyper-ramifié est dégradable par voie enzymatique.

27. Préparation selon au moins l'une des revendications précédentes, **caractérisée en ce qu'**au moins 80 % en poids des particules se situent dans une plage de taille de 100 µm ou moins.

28. Préparation selon au moins l'une des revendications précédentes, **caractérisée en ce que** le polymère hyper-ramifié présente à 48 °C dans un mélange à 50 % en poids/50 % en poids avec de l'eau seulement des équilibres liquide-liquide, mais ne présente pas d'équilibres solide-liquide ni d'équilibres solide-liquide-liquide.

29. Procédé pour la production de préparations selon au moins l'une des revendications 1 à 28, par mélange d'un polymère hyper-ramifié et d'au moins une substance de faible masse moléculaire.

30. Utilisation d'une préparation selon au moins l'une des revendications 1 à 28, en tant que durcisseur, en tant qu'agent de réticulation, en tant que catalyseur, en tant qu'antimousse, en tant que dispersant, en tant qu'additif à action antimicrobienne ou antifongicide, en tant qu'additif destiné à augmenter la résistance à la rayure de surfaces, en tant qu'additif dans des revêtements ou des adhésifs, en tant d'agent améliorant l'écoulement et/ou en tant qu'additif pour béton.

31. Utilisation d'une préparation selon au moins l'une des revendications 1 à 28 dans des cosmétiques, dans des médicaments, dans des déodorants, dans des produits alimentaires, dans des aliments pour animaux, dans des boissons, dans des revêtements de sols ou de murs, dans des matériaux de garnitures de joints, dans des emballages et/ou dans des systèmes de peintures.

32. Procédé pour la libération de substances de faible masse moléculaire à partir de préparations selon au moins l'une des revendications 1 à 28, **caractérisé en ce que** le polymère hyper-ramifié est dégradé par voie enzymatique.

33. Procédé pour la production de préparations selon au moins l'une des revendications 1 à 28, **caractérisé en ce qu'**un gaz comprimé est utilisé ou au moins deux phases liquides apparaissent, les deux phases contenant la substance de faible masse moléculaire et au moins une phase liquide contenant un polymère hyper-ramifié.

34. Procédé pour la production de préparations selon au moins l'une des revendications 1 à 28, comprenant les étapes de préparation d'une masse fondue de polymère comprenant au moins un polymère hyper-ramifié et au moins une substance de faible masse moléculaire, d'introduction de la masse fondue de polymère dans une deuxième phase liquide, dans laquelle le polymère hyper-ramifié est difficilement soluble et qui présente une température de solidification inférieure à la température de solidification du polymère hyper-ramifié, de dispersion de la masse fondue de polymère dans la deuxième phase liquide à une température qui est supérieure ou égale à la température de solidification du polymère hyper-ramifié et de solidification de la masse fondue de polymère dispersée dans la deuxième phase liquide.
